# FASCICULE DE BREVET EUROPEEN

(11) **EP 1 700 128 B1**
(45) Date de publication et mention de la délivrance du brevet: **12.08.2009**
(21) Numéro de dépôt: 04817587.1
(22) Date de dépôt: 23.12.2004
(51) Int. Cl.: G01N 33/569, G01N 33/543

(54) **METHODE DE DIAGNOSTIC SEROLOGIQUE ET DETERMINATION DE STATUT VACCINAL COMPRENANT DIFFERENTS CONTROLES**
VERFAHREN ZUR SEROLOGISCHEN DIAGNOSE UND BESTIMMUNG DES IMMUNISIERUNGSZUSTANDS UNTER EINSCHLUSS VERSCHIEDENER KONTROLLEN
METHOD FOR SEROLOGICAL DIAGNOSIS AND DETERMINATION OF IMMUNISATION STATUS, COMPRISING VARIOUS CONTROLS

(30) Priorité: 24.12.2003 FR 0315327; 03.08.2004 FR 0408600
(43) Date de publication de la demande: 13.09.2006
(73) Titulaire: Inodiag, 13600 la Ciotat (FR)
(72) Inventeur: ESCARGUEL, Claude, F-83110 Sanary-sur-Mer (FR)
(74) Mandataire: Domange, Maxime
(86) Numéro de dépôt international: PCT/FR2004/003364
(87) Numéro de publication internationale: WO 2005/064340

(56) Documents cités:
- EP-A- 0 453 060
- US-A1- 2003 017 616
- US-A1- 2003 175 699
- DATABASE WPI Section Ch, Week 200410 Derwent Publications Ltd., London, GB; Class B04, AN 2004-095735 XP002333639 & RU 2 213 972 C1 (VACCINE & SERUM INST) 10 octobre 2003 (2003-10-10)

## Description

La présente invention concerne une méthode et une trousse de diagnostic des maladies infectieuses humaines et animales. Plus particulièrement, elle concerne une méthode de diagnostic des maladies infectieuses humaines et animales qui repose sur la recherche dans le sérum du patient d'anticorps spécifiques de l'agent microbien infectieux, à savoir une bactérie, un virus, un parasite ou un champignon responsable d'une pathologie (désignés ci-dessous "microbe").

La présente invention concerne également une méthode et une trousse de diagnostic pour la détermination sérologique du statut vaccinal de personnes.

Dans la présente invention, le terme de "patient" est pris au sens large, incluant les patients humains et également les animaux. On entend, plus particulièrement, par "animal" :
- les oiseaux, plus particulièrement encore les volailles que l'on élève pour leurs oeufs ou leur chair, et
- les mammifères, notamment chiens, chats, chevaux, bovins tels que veaux, vaches , ovins tels que moutons, et caprins tels que chèvres.

Cette méthode de diagnostic est primordiale pour le diagnostic des infections à microbes, de prélèvement, de culture et/ou d'identification difficile, en particulier les virus et les bactéries intracellulaires strictes et les bactéries intracellulaires facultatives des genres *Rickettsia, Coxiella, Bartonella, Tropheryma, Ehrichia, Chlamydia, Mycoplasma, Treponema, Borrelia,* et *Leptospira* par exemple, pour lesquels on utilise, comme antigènes microbiens, des antigènes constitués de microbes entiers ou de fractions ou fragments de microbes comprenant un ou plusieurs antigènes. Il est en effet possible de fragmenter mécaniquement le microbe par agitation mécanique ou par sonication par exemple ou bien de fragmenter le microbe par un procédé enzymatique afin d'en obtenir une fraction conservant les antigènes qui supportent la réaction sérologique, objet de la présente invention. Les fractions ainsi obtenues sont séparées ou encore purifiées des autres constituant du microbe et de son milieu de culture par un procédé approprié, par exemple par centrifugation ou par filtration. On parle alors de "fraction antigénique" ou "d'antigène purifié". Le microbe entier ou une quelconque fraction du microbe est appelé ci-après "antigène particulaire ou corpusculaire" en ce que le microbe entier ou une de ses fractions ne peut pas être mis en solution par dissolution, mais uniquement en suspension dans un fluide approprié. Les microbes ou leur fraction restent visibles en tant que particules individualisables par observation microscopique à l'aide d'un microscope optique ou d'un microscope électronique par exemple.

Techniquement, la sérologie microbienne consiste à détecter dans le sérum du patient une réaction antigène - anticorps dans laquelle l'antigène est représenté par tout ou fraction de l'agent microbien infectieux à détecter et l'anticorps est représenté par les immunoglobulines humaines ou animales spécifiques dudit agent microbien infectieux présentes dans le sérum du patient. Elle peut être quantifiée en testant successivement une série de dilutions croissantes de raison 2 ou de raison 10 du sérum du patient à partir d'une première dilution au 1 :16 ou bien au 1 :50 du sérum du patient.

Dans la méthode de l'invention, on dépose donc, le cas échéant, l'antigène sur un support solide du type lame de verre, ou microplaque de titration pour mettre en oeuvre des techniques de détection par immunodétection, notamment immunofluorescence, ou par technique enzymatique, notamment du type ELISA. Ces techniques de détection sont bien connues de l'homme de l'art, et comprennent les étapes successives de :
1. décomplémentation du sérum par chauffage à 56°C pendant 30 minutes,
2. mise en contact de l'antigène correspondant à l'agent microbien infectieux fixé sur support solide, avec le sérum du patient puis incubation sous des conditions de temps, de température, d'hygrométrie, d'agitation mécanique et de force ionique du milieu permettant la réaction antigène - anticorps,
3. lavage précautionneux et extensif permettant d'éliminer le sérum du patient non-fixé au support solide, en excès,
4. application d'un anticorps secondaire de détection qui est une immunoglobuline animale dirigée contre les immunoglobulines de l'espèce du patient considéré, par exemple dans le cas d'un patient humain une immunoglobuline de chèvre anti-immunoglobuline humaine conjuguée à une substance fluorochrome, généralement de l'iso-thio-cyanate de fluoresceine ou une enzyme, généralement une peroxydase et incubation sous des conditions de temps, de température, d'hygrométrie, d'agitation mécanique et de force ionique du milieu permettant la réaction antigène - anticorps,
5. lavage précautionneux et extensif permettant d'éliminer l'immunoglobuline marquée, non fixée, en excès,
6. détection d'une réaction par lecture, à l'aide d'un appareillage approprié en fonction du marqueur tel qu'un microscope à fluorescence ou un lecteur de puces biologiques (microarray en anglais) pour la technique d'immunofluorescence indirecte, ou un lecteur de densité optique pour la technique de détection enzymatique du type ELISA. La lecture de la réaction se fait à l'oeil nu ou après acquisition numérique du gel et analyse par un logiciel de densitométrie ou tout logiciel approprié pour le traitement des images.

La présente invention concerne plus particulièrement les méthodes de détection et dosage dans lesquelles on détecte la présence et, de préférence, on dose la quantité d'immunoglobulines de classe M (IgM) et G(IgG) spécifiques d'un antigène microbien caractéristique d'un microbe. Ces déterminations donnent des informations plus précises et complètes, nécessaires pour établir l'étiologie et suivre l'évolution de certaines maladies infectieuses. Ainsi, en général les IgM apparaissent de façon plus précoce. Quant aux IgG, elles permettent d'établir le statut sérologique du patient vis à vis d'un microbe donné, en vue d'établir le diagnostic sérologique de l'infection ou d'établir le degré de protection de l'organisme contre ce microbe.

La spécificité de la réaction antigène infectieux/anticorps sérique conditionne la spécificité et la valeur prédictive positive du test sérologique basé sur cette réaction et toute fixation d'un anticorps non-spécifique sur l'antigène microbien limite la spécificité et la valeur prédictive du test sérologique. La présence dans le sérum à tester, de facteurs rhumatoïdes ou d'anticorps anti-nucléaires, est une source de fixation non-spécifique d'anticorps sur l'antigène microbien comme expliqué ci-après.

Ces facteurs rhumatoïdes sont des immunoglobulines de classe M reconnaissant le fragment Fc des IgG de différentes espèces dont les IgG humaines (IgM anti IgG).

La présence de facteurs rhumatoïdes dans le sérum d'un patient est responsable de résultats faussement positifs lors de la détection d'IgM spécifiques d'un antigène microbien au cours des tests sérologiques pour le diagnostic indirect des maladies infectieuses. En effet, ces facteurs rhumatoïdes se fixent aux IgG spécifiques de l'antigène microbien contenu dans le sérum du patient, et apparaissent donc faussement positifs lors de la détection des IgM spécifiques dans la réaction sérologique utilisant l'antigène microbien dans des tests de détection par agglutination.

Il est possible d'éliminer les facteurs rhumatoïdes du sérum par une adsorption par des IgG humaines non spécifiques que l'on introduit en excès dans l'échantillon. Ces IgG introduites sont modifiées par chauffage ou traitement par le glutaraldéhyde de façon à ce qu'elles n'interfèrent pas avec la détection des IgG spécifiques. Cet ajout d'IgG provoque une précipitation après complexation avec les substances anti IgG contenues dans l'échantillon. Mais, dans ce cas, les IgG et IgM spécifiques contenues dans l'échantillon peuvent aussi être dénaturées par le traitement de modification des IgG introduites dans l'échantillon.

D'autre part, il n'existe pas, à ce jour, de contrôle systématique de l'efficacité de cette adsorption avant la mise en oeuvre de la réaction sérologique dans le cadre d'une méthode de détection et de dosage d'IgM et d'IgG..

De même, la présence dans le sérum du patient d'anticorps anti-nucléaires limite la spécificité de la réaction antigène microbien-anticorps sériques. Les anticorps anti-nucléaires sont en effet des anticorps du type IgG dirigés de façon non-spécifique contre l'ensemble formé par l'ADN et les protéines nucléaires du chromosome des cellules eucaryotes et des microbes, appelées histones. Ces anticorps anti-nucléaires se fixent donc de façon non-spécifique sur toute cellule eucaryote y compris les champignons et les parasites et sur tout microbe, bactérie, virus à ADN, parasite ou champignon. Ce phénomène entraîne une réaction positive non-spécifique au cours des tests sérologiques utilisant la détection d'IgG spécifique d'une bactérie, un virus à ADN, un champignon ou un parasite entier ou comprenant des complexes ADN/histones comme antigène microbien à l'aide d'anticorps de détection anti IgG dans un sérum contenant des anticorps anti-nucléaires.

La détection des anticorps anti-nucléaires dans le sérum à tester, réalisée en utilisant une technique d'immunodétection par immunofluorescence, a été décrite [Fritzler M.J. In : Manual of Clinical Laboratory Immunology, Fourth edition, Rose N.R., Conway de Macario E., Fahey J.L., Friedman H., Penn, G.M., (eds.). American Society for Microbiology, Washington, D.C. 1992, pp. 724-729] utilisant des cellules mammifères y compris des cellules humaines telles que des cellules Hep-2 comme antigène. Dans cette méthode, les cellules sont constituées par un tapis de cellules confluentes déposées et « lues » manuellement. Ce dépôt de cellules confluentes est trop visqueux pour être robotisable par dépôt avec un robot de type seringue, et la lecture n'est pas automatisable car, seul, le dépôt manuel permet de déposer une grande quantité de cellules de manière à garantir une détection suffisante de la réaction Cellule-anticorps anti-nucléaires. Dans ces publications, on ne mentionne pas la nécessité de réaliser un contrôle systématique de la présence d'anticorps anti-nucléaires dans des tests d'immunodétection d'antigènes microbiens. En outre, la détection avec des cellules confluentes déposées manuellement sur support solide n'est pas applicable pour des tests de routine de laboratoire.

Il n'y a donc pas de méthode publiée à ce jour, pour le contrôle ou l'élimination des anticorps anti-nucléaires avant la réalisation d'un test sérologique pour le diagnostic des maladies infectieuses de façon à garantir lors du résultat l'absence d'une réaction faussement positive, applicable en routine de laboratoire. C'est pourquoi, aucun protocole de sérologie publié dans les manuels de référence ni aucun test sérologique commercialisé n'inclut systématiquement le dépistage d'anticorps anti-nucléaires ni de facteur rhumatoïde comme préalable à la réalisation ou à l'interprétation de la sérologie.

En pratique, à ce jour, dans les tests d'immunodétection en routine de laboratoire, on ne peut, tout au plus, détecter les faux positifs dus à la présence de facteur rhumatoïde (ou anticorps anti-nucléaires) qu'en effectuant des tests sur une pluralité d'antigènes microbiens dont la présence concomitante n'est pas vraisemblable. Mais, ce type de vérification, on le comprend, augmente considérablement le coût en terme de matériel et main d'oeuvre ainsi que la durée des tests.

On connaît des méthodes et kit de diagnostic impliquant l'utilisation d'un support solide sur lequel sont fixés de façon covalente des protéines solubles, mais ces couplages chimiques covalents sont complexes et coûteux à réaliser. On a proposé la fixation non covalente de protéine soluble ou antigène particulaire sur support solide par adsorption physique ou physico-chimique sur le support, dans le cadre des protocoles de tests d'immunodétection sur support solide, mais la stabilité de la fixation est insuffisante. Une difficulté tient à ce qu'il est nécessaire de bien laver préalablement le support solide pour éliminer les résidus d'éléments de marquage pouvant interférer avec la lecture des résultats alors que ces lavages rendent l'adsorption physique des substances sur le support solide trop instable. Une autre difficulté tient à ce qu'il est pas possible de déposer avec des robots de dépôts des antigènes corpusculaires qu'il s'agisse de cellules ou bactéries entières ou partielles comme mentionné ci-dessus.

US 2003/0017616 décrit des phases solides sur lesquelles sont fixés des réactifs de détection d'antigènes ou d'anticorps dans un échantillon de sérum humain et des réactifs de contrôle de réactions non spécifiques.

Un premier but de la présente invention est de fournir un test fiable d'immunodétection d'antigènes microbiens, par une méthode et des outils simples à mettre en oeuvre et réaliser pour une application en routine de laboratoire dans le cadre de la réalisation des tests en série, dans le cas où le sérum testé pourrait contenir des facteurs rhumatoïdes ou des anticorps anti-nucléaires, et une méthode qui permette de s'affranchir de la mise en oeuvre d'échantillons de sérums de contrôle positif et/ou négatif.

Un autre but de la présente invention est de fournir une méthode de préparation de support solide permettant un dépôt robotisé et fixation non covalente par adsorption physique sur le support d'antigènes témoins de la présence de facteur rhumatoïde et anticorps anti-nucléaire et antigène microbien, constitués de protéines ou d'antigènes particulaires comme expliqué ci-dessus, ainsi que la lecture automatisée du contrôle d'absence de facteur rhumatoïde et d'anticorps anti-nucléaires et du résultat de la réaction immunologique dans un test de sérum de patient impliquant une réaction de sérologie microbienne des IgM ou bien des IgG spécifiques par adsorption avec l'antigène microbien particulaire ou corpusculaire déposé sur support solide.

De même, aucun test sérologique commercialisé n'inclut systématiquement le contrôle de l'introduction et de la valeur réactive (ou réactivité immunologique) des anticorps secondaires de détection anti IgM ou anti IgG mis en oeuvre, et un autre but original de la présente invention est de fournir un test comprenant ce contrôle de réactivité.

Pour ce faire, la présente invention fournit une méthode de diagnostic sérologique in vitro d'agent microbien par immunodétection dans laquelle on détecte la présence et, de préférence, on dose la quantité d'immunoglobulines de patient des deux classes M et G ou seulement de la classe G spécifiques d'un antigène microbien caractéristique dudit agent microbien, dans un échantillon de sérum du patient à tester, par détection et quantification d'un complexe de réaction immunologique entre ledit antigène microbien à détecter et une dite immunoglobuline spécifique de classe G pour le dosage d'IgG, ou de classe M pour le dosage d'IgM, à l'aide d'une première substance de détection constituée par un anticorps ne réagissant qu'avec une dite immunoglobuline de l'espèce du patient de classe G pour le dosage d'IgG, ou une seconde substance de détection constituée d'un anticorps ne réagissant qu'avec une dite immunoglobuline de l'espèce du patient de classe M pour le dosage d'IgM, **caractérisée en ce que :**
1/- on réalise les étapes dans lesquelles :
   ■ on met en contact ledit échantillon de sérum à tester puis lesdites première et seconde substances de détection ou seulement ladite première substance de détection avec au moins un support solide sur lequel ont été fixés les antigènes suivants :
      - un premier antigène de contrôle correspondant à une immunoglobuline non spécifique de classe G de l'espèce du patient, et
      - un deuxième antigène de contrôle comprenant des complexes ADN/histones, de préférence tout ou partie de cellules nucléées comprenant des noyaux de cellules nucléées, de préférence encore des cellules en lignée continue, de préférence encore des cellules non confluentes en suspension, et
      - en cas de dosage d'IgM, un troisième antigène de contrôle correspondant à une immunoglobuline non spécifique de classe M de l'espèce du patient; la présence dudit troisième antigène de contrôle étant nécessaire, et
      - au moins un dit antigène microbien, et
   ■ on réalise une série de contrôles comprenant:
      a- le contrôle de la réactivité de ladite première substance de détection en vérifiant si ledit premier antigène de contrôle réagit avec ladite première substance de détection, et, en cas de dosage d'IgM, le contrôle de la présence de facteurs rhumatoïdes dans ledit échantillon de sérum en vérifiant si le premier antigène de contrôle réagit avec ledit échantillon de sérum et ladite seconde substance de détection,
      b- le contrôle de la présence d'anticorps antinucléaires dans ledit échantillon de sérum à tester en vérifiant si ledit deuxième antigène de contrôle réagit avec ledit échantillon de sérum et la première substance de détection,
      c- le contrôle de la réactivité de ladite seconde substance de détection en vérifiant si ledit troisième antigène de contrôle réagit avec ladite seconde substance de détection, en cas de dosage d'IgM, et
      d- le contrôle de la présence d'un sérum de l'espèce du patient dans l'échantillon à tester, et
2/- on ne prend en compte le résultat d'une réaction entre ledit antigène microbien, ledit échantillon de sérum et une dite substance de détection que si le contrôle de la présence d'un sérum de l'espèce du patient est positif et si les conditions cumulatives suivantes sont réunies, établissant l'absence d'anticorps antinucléaire et la réactivité desdites première et, le cas échéant, seconde substances de détection et le cas échéant de facteur rhumatoïde:
   a- ledit premier antigène de contrôle réagit avec ladite première substance de détection,
   b- ledit deuxième antigène de contrôle ne réagit pas, et
   c- le cas échéant, ledit troisième antigène de contrôle réagit avec ladite seconde substance de détection, en cas de dosage d'IgM.

Si l'échantillon comprend des facteurs rhumatoïdes (IgM anti IgG), ceux-ci peuvent réagir avec les immunoglobulines (IgG₁) fixées sur le support solide, pour former le complexe suivant avec ladite seconde substance de détection (Ac₁ anti IgM*¹) : (S-IgG₁-IgM anti IgG - Ac₁ anti IgM*¹) (S= support solide).

Quand l'absence de facteur rhumatoïde est établie, la détection d'une réaction entre ladite seconde substance de détection et ledit antigène microbien (Agmic) est effectivement la preuve de la présence dans le sérum testé d'immunoglobulines de classe M spécifique de l'antigène microbien (IgM anti Agmic) par formation du complexe (S-Agmic-IgM anti Agmic-Ac₁ anti IgM*¹), et non pas de la présence d'IgG spécifique du dit antigène microbien (IgG anti Agmic), lesquels pourraient en effet former, en présence de facteur rhumatoïde, un complexe faux positif (S-Agmic-IgG anti Agmic-IgM anti IgG - Ac₁ anti IgM*¹).

En outre comme explicité ci-après, lorsque ledit premier antigène est constitué d'une IgG de l'espèce du patient notamment humaine, ceci permet de vérifier la présence et la réactivité d'une dite première substance de détection reconnaissant spécifiquement les IgG du sérum de l'espèce du patient.

Dans le cas d'un patient humain, on peut utiliser avantageusement, comme dit deuxième antigène de contrôle, des cellules de fibroblastes humains, notamment des cellules HL60.

Si l'échantillon à tester comprend des anticorps anti-nucléaires (qui sont des IgG, notamment humaines), ceux-ci peuvent réagir avec ledit deuxième antigène (Ag₂) et être détectés par ladite première substance de détection (Ac₂ anti IgG*²) puisque celle-ci est une substance réagissant avec des IgG de l'espèce du patient, notamment humaines, et former le complexe (S-Ag₂-IgG anti Nucl- Ac₂ anti IgG*²).

La détection d'une réaction entre ladite seconde substance de détection et ledit deuxième antigène (Ag₂) fixé sur support solide, signifie nécessairement que s'est formé un complexe avec des anticorps anti-nucléaires (Ac anti nucl.) suivant :(S-Ag₂-IgG anti nucl.-IgM anti IgG- Ac₁ anti IgM*¹), et donc que l'échantillon comprend à la fois le facteur rhumatoïde et des anticorps anti-nucléaire.

Une fois l'absence d'anticorps anti-nucléaire établie, la détection d'une réaction de ladite première substance de marquage avec ledit antigène microbien est bien la preuve de la présence d'IgG spécifique du dit antigène microbien et de formation d'un complexe (S-Agmic-IgG antiAgmic-Ac₂ anti IgG*²) et non pas d'un complexe faux positif résultant de la réaction des anticorps anti-nucléaires avec l'antigène microbien selon le complexe (S-Agmic-Ac anti nucl.-Ac₂ anti IgG*²).

On vérifie aussi la réactivité de ladite première substance de détection introduite dans ledit échantillon de sérum à tester, en l'absence de facteur rhumatoïde et d'anticorps anti-nucléaires dans ledit échantillon. En effet, si ladite première substance de détection est bien réactive en l'absence de facteur rhumatoïde, on doit détecter le complexe suivant (S-IgG₁-Ac₂ anti IgG*²) sur ledit premier antigène (IgG₁). Dans ce cas, l'absence de réaction de ladite première substance de détection avec ledit deuxième antigène est bien la preuve de l'absence d'anticorps anti-nucléaires.

Si ladite seconde substance de détection est présente et est bien réactive, on doit détecter un complexe S-IgM₁-Ac₁ anti IgM*¹ sur ledit troisième antigène de contrôle (IgM₁). Dès lors, l'absence de détection d'un complexe comprenant ledit second marqueur au niveau du dit premier antigène (IgG₁) et, le cas échéant, au niveau du dit second antigène fixé sur support solide, est bien la preuve de l'absence de facteur rhumatoïde.

Plus particulièrement, on effectue un dépôt robotisé d'une solution d'IgG et d'IgM de l'espèce du patient, notamment humaines, γ-spécifique (spécifique de la chaîne gamma des immunoglobulines de l'espèce du patient, notamment humaines) comme dits premier et troisième antigènes de contrôle.

La présente invention permet donc les détections systématiques de facteurs rhumatoïdes, d'anticorps anti-nucléaires, et le contrôle systématique de la réactivité des anticorps anti immunoglobulines de détection dans un sérum utilisé pour un diagnostic sérologique par immunofluorescence indirecte, après le dépôt robotisé sur support solide d'immunoglobulines de l'espèce du patient, notamment humaines, de classe G et M et de cellules nucléées de l'espèce du patient.

Une autre erreur fréquente dans la réalisation des tests sérologiques, notamment pour les tests sérologiques en batterie réalisés sur un grand nombre de sérums à tester, est due à des défauts dans l'introduction des sérums à tester, notamment par pipetage. Ces erreurs interviennent notamment dans les étapes qui impliquent le déplacement de l'échantillon à tester, notamment par pipetage, certains récipients, notamment contenant le support solide sur lequel est déposé l'antigène à détecter, peuvent ne pas être remplis par inadvertance avec le sérum de l'espèce du patient, notamment humaine, à tester. Il est connu que le pipetage de sérum est entaché d'un risque d'erreurs de 1‰, liée à un problème purement technique par absence de pipetage par la pipette, ou à une erreur humaine par absence de pipetage par inadvertance

Ces erreurs imposent l'introduction de contrôles dans la réalisation de la réaction. L'incorporation systématique au cours de chaque nouvelle manipulation d'un sérum témoin négatif c'est à dire ne contenant pas d'anticorps spécifiques de l'antigène à tester permet d'interpréter les réactions positives. De même, l'incorporation d'un sérum témoin positif, c'est à dire contenant l'anticorps spécifique de l'antigène testé à un titre connu permet de vérifier la qualité de l'antigène et de l'immunoglobuline conjuguée.

Cependant, il n'existe actuellement aucun contrôle de ce que le sérum à tester a bien été introduit dans le test sérologique. Or, si par inadvertance, le sérum à tester n'est pas introduit dans le test sérologique, la réaction antigène bactérien-anticorps sérique n'existera certainement pas, et le test sera interprété, faussement, comme négatif (faux-négatif). Dans la présente invention, on tire partie de ce que la protéine A du *Staphylococcus aureus* (staphylocoque doré) présente une affinité pour les sérums d'origine animale, notamment le cheval, les bovins, le cochon, le lapin, le cobaye, la souris ; à un moindre degré le hamster, le rat et le mouton. En revanche, le sérum de poussin et de chèvre ne réagisse pas avec la protéine A. La protéine A est un polypeptide de 42 kDa, et est un constituant de la paroi des souches de *Staphylococcus aureus,* des protéines similaires mais différentes sont caractérisées à la surface des bactéries du genre *Streptococcus* [Langone J.J. Adv. Immunol. 1982, 32 : 157-251]. Cette propriété de la protéine A de *Staphylococcus aureus,* a déjà été utilisée dans un test sérologique chez l'homme pour le diagnostic sérologique des endocardites infectieuses [Rolain JM, Lecam C, Raoult D. Simplified serological diagnosis of endocarditis due to Coxiella burnetii and Bartonella. Clin. Diag. Lab. Immunol. 2003 ; 10 :1147-8].

La présente invention comprend donc l'introduction d'un contrôle d'introduction du sérum à tester au cours des réactions de sérologies bactériennes.

Dans la mesure où la protéine A réagit avec des immunoglobulines animales et humaines de façon non spécifique, et ce même en cas de pathologie infectieuse importante, il est possible d'utiliser cette protéine A comme contrôle positif de l'introduction d'un sérum de l'espèce du patient, notamment humaine, dans l'échantillon à tester.

Plus précisément, dans la présente invention, on contrôle que ledit échantillon testé contient bien un sérum de l'espèce du patient en détectant si des immunoglobulines de l'espèce du patient réagissent avec un quatrième antigène de contrôle contenant la protéine A d'une bactérie *Staphylococcus aureus,* de préférence ledit quatrième antigène étant une bactérie *Staphylococcus* entière, en mettant en contact ledit échantillon avec un support solide sur lequel est fixé un dit quatrième antigène de contrôle, en présence de ladite première substance de détection qui est une substance réagissant avec une immunoglobuline de l'espèce du patient et ne réagissant pas avec ledit quatrième antigène de contrôle, de préférence un anticorps anti-immunoglobuline de l'espèce du patient ne réagissant pas avec ledit quatrième antigène de contrôle, le contrôle de la présence d'un sérum étant positif si ledit quatrième antigène réagit avec ledit échantillon de sérum et ladite première substance de détection.

Dans un mode de réalisation avantageux, ledit quatrième antigène est une bactérie entière *Staphylococcus aureus* comprenant la protéine A. On peut plus particulièrement utiliser les bactéries *Staphylococcus aureus* déposées dans les collections publiques telles que les bactéries déposées à l'A.T.C.C. sous le N°29213 et à la C.N.C.M. de l'Institut Pasteur (France) sous le numéro 65.8T comme décrit dans la publication mentionnée ci-dessus [Rolain JM, Lecam C, Raoult D. Simplified serological diagnosis of endocarditis due to Coxiella burnetii and Bartonella. Clin. Diag. Lab. Immunol. 2003 ; 10 :1147-8].

Par ailleurs, en dehors des souches-types, toute souche bactérienne identifiée comme *Staphylococcus aureus* peut être utilisée comme dit quatrième antigène de contrôle.

De préférence, ladite seconde substance de détection, est une immunoglobuline animale, de préférence une immunoglobuline de chèvre ou de poulet.

Avantageusement, on utilise un unique support solide mis en contact avec le cas échéant simultanément desdites première et seconde substances de détection comprenant un premier et respectivement un second élément de marquage, le second élément de marquage émettant un signal différent du premier élément de marquage, de préférence lesdites première et seconde substances de détection comprenant un premier et respectivement un second anticorps ne réagissant qu'avec une dite immunoglobuline de l'espèce du patient de classe G et respectivement de classe M.

Avantageusement encore, le cas échéant, les deux dites première et seconde substances de détection sont des immunoglobulines de chèvre ou de poulet, respectivement anti- IgG et anti- IgM.

Avantageusement encore, la (ou les) dite(s) substance(s) de détection est (ou sont) un (ou des) anticorps conjugué(s) à un élément de marquage comprenant un (ou des) substance(s) fluorescente(s).Et, en particulier, on vérifie si l'on détecte par fluorescence un complexe produit de réaction entre ledit quatrième antigène et ladite troisième substance de détection.

Avantageusement, dans une méthode de diagnostic selon l'invention, on détecte et, le cas échéant, on quantifie la dose de dite immunoglobuline de l'espèce du patient spécifique du dit antigène microbien dans l'échantillon à tester, et les réactions immunologiques entre lesdits antigènes de contrôle et lesdites substances de détection, par lecture automatisée à l'aide d'un appareil de lecture approprié aux dits éléments de marquage, de préférence un appareil de lecture d'un signal fluorescent d'une substance fluorescente correspondant aux éléments de marquage desdites substances de détection.

Comme élément de marquage des dites substances de détection, on utilise donc avantageusement un marquage enzymatique, radioactif ou fluorescent, ce dernier type de marquage fluorescent étant préféré.

L'expression « marquage fluorescent » signifie que l'anticorps a été rendu fluorescent par couplage ou complexation avec un agent fluorescent approprié tel que l'iso(thio)cyanate de fluorescéine ou toute autre substance émettant un rayonnement détectable après son illumination, chaque substance étant caractérisée par la longueur d'onde à laquelle elle doit être illuminée (longueur d'onde d'excitation) et la longueur d'onde du rayonnement qu'elle émet (longueur d'onde d'émission).

L'expression « marquage radioactif » signifie que l'anticorps porte un isotope radioactif permettant de le doser par comptage de la radioactivité qui lui est associée, l'isotope pouvant être porté soit sur un élément de la structure de l'anticorps, par exemple les résidus de tyrosine constitutifs, soit sur un radical approprié qui lui a été fixé.

L'expression « marquage enzymatique » signifie que l'anticorps spécifique est couplé ou complexé à une enzyme qui, associée à l'emploi de réactifs appropriés, permet une mesure quantitative de cet anticorps spécifique.

Le substrat et les réactifs sont choisis de sorte que le produit final de la réaction ou de la séquence de réactions provoquée par l'enzyme et mettant en oeuvre ces substances, soit :
- ou bien une substance colorée ou fluorescente qui diffuse dans le milieu liquide environnant l'échantillon testé et qui fait l'objet, soit de la mesure finale spectrophotométrique ou fluorimétrique, respectivement, soit d'une évaluation à l'oeil ; éventuellement par rapport à une gamme de teintes étalonnées,
- ou bien une substance colorée insoluble qui se dépose sur l'échantillon testé et qui peut faire l'objet, soit d'une mesure photométrique par réflexion, soit d'une évaluation à l'oeil, éventuellement par rapport à une gamme de teintes étalonnées.

Lorsque l'on utilise une substance de détection rendue fluorescente, la fluorescence associée à l'échantillon testé est lue directement sur un appareil approprié capable de détecter le rayonnement à la longueur d'onde d'émission et de le quantifier.

Selon un mode de réalisation avantageux, lesdits antigènes de contrôle et antigènes microbiens sont fixés sur support solide par adsorption physique. Et, avantageusement encore, ledit antigène microbien est un antigène particulaire ou corpusculaire tel que décrit précédemment, constitué de microbe entier inactivé ou fraction de microbe.

Comme explicité ci-après, les inventeurs ont en effet élaboré des conditions de dépôt sur support solide desdits antigènes de contrôle et microbiens qui permettent de s'accommoder d'un dépôt par simple adsorption physique, et ce plus particulièrement lorsque lesdits antigènes microbiens sont des antigènes corpusculaires.

Dans un mode de réalisation particulier, ledit antigène microbien est choisi parmi des micro-organismes comprenant une bactérie, un virus, un parasite ou un champignon.

Plus particulièrement, ledit antigène microbien est une bactérie intracellulaire, et notamment, ledit antigène microbien est choisi parmi les bactéries du genre *Rickettsia, Coxiella, Bartonella, Tropheryma, Ehrlichia, Chlamydia, Mycoplasma, Treponema, Borrelia,* et *Leptospira,* cette liste n'étant pas exhaustive.

Plus particulièrement encore, ledit antigène microbien est une bactérie responsable d'une endocardite.

Dans un autre mode de réalisation, ledit antigène est un antigène microbien est un antigène viral, notamment un virus choisi parmi les virus H.I.V., C.M.V. ou Epstein-Barr, cette liste n'étant pas exhaustive.

De préférence, une méthode selon l'invention comprend les caractéristiques selon lesquelles :
- on détecte et, de préférence, on dose la quantité des immunoglobulines de patients des deux classes M et G spécifiques d'un antigène microbien,
- au moins un dit antigène microbien et lesdits premier, deuxième et troisième et quatrième antigènes de contrôle sont fixés sur un même support solide et
- on utilise pour la détection des différents dits antigènes microbiens, les mêmes dites première et seconde substances de détection avec des éléments de marquage différents, lesdites première et deuxième substances de détection étant des immunoglobulines animales ne réagissant pas avec ledit quatrième antigène, de préférence dans le cas d'un patient humain, des immunoglobulines de chèvre ou de poulet.

Dans ce dernier mode de réalisation, un protocole de succession des contrôles est le suivant :
1) On vérifie que ledit quatrième antigène de contrôle contenant la protéine A réagit avec ladite première substance de détection. A défaut, on arrête le test, c'est-à-dire on ne prend pas en compte cet échantillon.
2) Si ledit premier antigène de contrôle (IgG₁) réagit avec ladite seconde substance de détection (Ac1 anti IgM^{*1}), l'échantillon de sérum comprend des facteurs rhumatoïdes, donc, là encore, on ne prend pas en compte les tests de détection et de quantification des IgM spécifiques.
3) Si ledit premier antigène de contrôle ne réagit pas avec la première substance de détection (Ac₂ anti IgG*²), ladite première substance de détection n'est pas présente ou pas réactive. On ne prend pas en compte le résultat du test concernant la détection des IgG spécifiques de l'antigène microbien.
4) Si ledit premier antigène de contrôle réagit avec la première substance de détection, il n'y a pas de facteur rhumatoïde, on peut continuer le test, c'est-à-dire prendre en compte les résultats sous réserve des vérifications suivantes concernant les réactions avec les deuxième, troisième et quatrième antigènes de contrôle.
5) Si ledit deuxième antigène de contrôle contenant un complexe ADN/histone réagit avec ladite première substance de détection, des anticorps antinucléaires sont présents et on ne prend pas en compte les tests de détection et de quantification des IgG spécifiques.
6) Si le dit deuxième antigène de contrôle réagit avec la dite deuxième substance de détection, des facteurs rhumatoïdes et des anticorps antinucléaires sont présents et on arrête le test.
7) Si ledit deuxième antigène de contrôle ne réagit pas, et que lesdites première et deuxième substances de détection sont présentes et réactives, il n'y a pas d'anticorps anti-nucléaire, et on peut continuer sous réserve de la vérification suivante.
8) On vérifie que ledit troisième antigène de contrôle réagit avec ladite seconde substance de détection. Si ledit troisième antigène de contrôle (IgM) ne réagit pas, ladite seconde substance de détection n'est pas présente ou ne réagit pas, et on arrête le test.

En résumé, on ne prend en compte le résultat de la réaction avec ledit antigène microbien, que si les conditions cumulatives suivantes sont réunies :
- ledit quatrième antigène réagit,
- ledit premier antigène réagit avec ladite première substance de détection,
- ledit deuxième antigène ne réagit pas, et
- ledit troisième antigène réagit avec ladite seconde substance de détection.

Dans un mode de réalisation particulier, ledit antigène microbien est un antigène vaccinal et ladite immunoglobuline spécifique du dit agent vaccinal à détecter est une immunoglobuline de classe G.

La méthode permet alors de déterminer le statut vaccinal d'un individu, ce qui est particulièrement utile pour apprécier la nécessité d'effectuer un rappel vaccinal.

La détermination dans le sérum d'une personne à vacciner, de la présence voire du taux d'anticorps protecteurs spécifiques sont un élément clé pour décider l'administration du vaccin ou du rappel vaccinal. En effet, si le sérum de la personne contient des anticorps spécifiques du pathogène à une concentration protectrice, il n'y a aucun bénéfice mais au contraire une prise de risque à administrer un rappel vaccinal à cette personne.

Dans les méthodes proposées actuellement, la détection de la présence ou la mesure de la concentration des anticorps spécifiquement dirigés contre les différents antigènes vaccinaux sont réalisées séparément les unes des autres. Il faut donc réaliser plusieurs prélèvements de sérum qui sont analysés séparément, le cas échéant dans plusieurs laboratoires disposant chacun d'une capacité d'analyse d'un seul antigène vaccinal ou bien d'un nombre restreint d'antigènes vaccinaux.

Il n'existe pas actuellement de trousse, ni de test automatisé et reproductible, permettant la détermination du statut vaccinal d'une personne par la détermination du titre d'anticorps spécifiques contre les principaux pathogènes vaccinaux. Ainsi, il n'est pas fourni actuellement de méthode et de kit permettant de déterminer dans un laps de temps inférieur à 24 heures sur un seul prélèvement de sérum, le statut vaccinal d'une personne, c'est-à-dire la détection ou la détermination des concentrations d'anticorps de classe IgG spécifiques en liaison avec l'ensemble des vaccins actuellement disponibles.

Un autre but de la présente invention est de fournir une technique de détermination du statut vaccinal d'une personne, qui soit simple, rapide et peu coûteuse, utilisable notamment par tout laboratoire pour connaître simultanément par analyse d'un même échantillon de sérum à tester, la concentration d'anticorps contre une pluralité de vaccins actuellement disponibles, et ce sur un volume d'échantillons relativement réduit et donc compatible pour des prélèvements chez l'enfant, y compris le nourrisson. Plus particulièrement encore, la technique de diagnostic doit pouvoir être automatisable et reproductible de façon fiable, aussi bien dans sa mise en oeuvre que dans la préparation des supports solides mis en oeuvre.

Pour ce faire, la présente invention fournit une méthode dans laquelle on détermine le statut vaccinal d'un individu par détection, de préférence quantification des anticorps sériques IgG spécifiques d'antigènes vaccinaux d'une pluralité d'agents pathogènes du type bactéries, virus, champignons ou parasites, en réalisant la détection, et de préférence la quantification, d'un complexe de réactions immunologiques entre chaque dit antigène vaccinal et respectivement chaque dit anticorps spécifique dudit antigène vaccinal, éventuellement présent dans un échantillon de sérum humain à tester, comprenant:
1- la mise en contact d'un seul et même dit échantillon de sérum à tester puis d'au moins une dite première substance de détection réagissant avec au moins un dit anticorps spécifique et ne réagissant pas avec l'un quelconque desdits antigènes vaccinaux, avec un même support solide sur lequel est fixé une pluralité de dits antigènes vaccinaux correspondant à une pluralité d'agents pathogènes, et lesdits premier, deuxième et de préférence quatrième antigènes de contrôle, et
2- au moins un dit contrôle de la réactivité de ladite première substance de détection à l'aide d'un dit premier antigène de contrôle et un dit contrôle de la présence d'anticorps antinucléaires à l'aide d'au moins un dit deuxième antigène de contrôle, et le contrôle de la présence d'un sérum humain dans ledit échantillon à tester, de préférence à l'aide d'un dit quatrième antigène de contrôle.

De façon connue également, la quantification est réalisée par comparaison du signal émis par le complexe de réaction antigène-anticorps-substance de détection du sérum testé avec une courbe de référence obtenue par calibration à l'aide de sérums témoins contenant une concentration connue d'anticorps à détecter.

On entend par "antigène vaccinal", un antigène susceptible de stimuler une réponse immunitaire du patient induisant la production d'anticorps sériques protecteurs, c'est à dire un anticorps se liant à l'agent pathogène de telle façon que l'organisme se trouve ainsi protégé contre les effets pathogènes dudit agent.

De préférence, dans la méthode selon l'invention, on utilise une même dite première substance de détection pour la détection des différents anticorps spécifiques des différents antigènes vaccinaux.

De préférence encore, et plus particulièrement, on met en oeuvre une dite première substance de détection qui est une immunoglobuline anti-IgG, de préférence une immunoglobuline de chèvre ou de poulet.

Dans un mode particulier de réalisation, lesdits antigènes vaccinaux sont des antigènes des agents pathogènes choisis parmi les virus des oreillons, de la rubéole, de la rougeole, de la varicelle, de la poliomyélite, de la fièvre jaune, de l'encéphalite à tique, de l'hépatite A, de l'hépatite B, et les bactéries de la coqueluche Bordetella pertussis, du tétanos et de la diphtérie.

Avantageusement, on détermine si la concentration de dit anticorps spécifiques atteint un seuil à partir duquel ledit anticorps spécifique a une action protectrice protégeant contre la maladie déterminée par le pathogène.

Dans certains cas, il est en effet possible de déterminer une concentration d'anticorps spécifiques conférant une protection dans près de 100% des personnes vaccinées, la détermination de cette concentration (ou seuil) est faite par des études séro-épidémiologiques sur de vastes populations.

Les méthodes selon l'invention sont plus particulièrement avantageuses lorsque qu'au moins un dit antigène microbien ou vaccinal est un antigène microbien corpusculaire constitué de microbe entier inactivé ou fraction de microbe, notamment un antigène vaccinal sous forme de suspension virale de virus entiers vivants désactivés.

Les inventeurs ont en effet développé une technologie par laquelle ce type d'antigène particulaire ou corpusculaire peut être fixé sur le support solide par simple dépose et adsorption physique ou liaison physico-chimique avec le support par une méthode de préparation de support solide selon l'invention comme il sera explicité ci-après. Ces antigènes particulaires ou corpusculaires ont l'avantage d'être particulièrement bien visibles après dépôt sur le support solide et, notamment, lors de la lecture de détection de réactions immunologiques éventuelles.

On utilise donc avantageusement, comme antigènes microbiens ou vaccinaux, des antigènes constitués de microbes entiers inactivés ou de fractions ou fragments de microbes comprenant un ou plusieurs antigènes. Il est en effet possible de fragmenter mécaniquement le microbe par agitation mécanique ou par sonication par exemple ou bien de fragmenter le microbe par un procédé enzymatique afin d'en obtenir une fraction conservant les antigènes qui supportent la réaction sérologique, objet de la présente invention. Les fractions ainsi obtenues sont séparées ou encore purifiées des autres constituant du microbe et de son milieu de culture par un procédé approprié, par exemple par centrifugation ou par filtration. On parle alors de fraction antigénique ou d'antigène purifié. Le microbe entier ou une quelconque fraction du microbe est appelé ci-après "antigène particulaire ou corpusculaire" en ce que le microbe entier ou une de ses fractions ne peut pas être mis en solution par dissolution, mais uniquement en suspension dans un fluide approprié.

Les microbes ou leur fraction ainsi déposés sont remarquablement nettement visibles en tant que particules individualisées par observation microscopique à l'aide d'un microscope optique ou d'un microscope électronique par exemple.

Techniquement, la sérologie microbienne consiste à détecter dans le sérum du patient une réaction antigène - anticorps dans laquelle l'antigène est représenté par tout ou fraction de l'agent microbien infectieux à détecter et l'anticorps est représenté par les immunoglobulines humaines ou animales spécifiques dudit agent microbien infectieux présentes dans le sérum du patient. Elle peut être quantifiée en testant successivement une série de dilutions croissantes de raison 2 ou de raison 10 du sérum du patient à partir d'une première dilution au 1/16 ou bien au 1/50 du sérum du patient.

Dans un mode préféré de réalisation, pour chaque détection et, le cas échéant, quantification d'un dit antigène vaccinal, on réalise les mesures suivantes :
1- une première mesure d'une première valeur représentative de la quantité d'un troisième élément de marquage ou "marqueur", de préférence la première valeur de l'intensité d'un signal émis par ledit troisième élément de marquage, de préférence encore fluorescent, ledit troisième élément de marquage se fixant de manière non spécifique sur toute protéine dans la zone de dépôt dudit antigène vaccinal, et
2- une deuxième mesure d'une deuxième valeur représentative de la quantité d'un premier élément de marquage émettant un signal différent dudit troisième élément de marquage et émettant un signal différent, de préférence une deuxième valeur de l'intensité du signal émis par ce troisième élément de marquage, de préférence encore fluorescent à une longueur d'onde d'excitation différente de celle dudit troisième élément de marquage fluorescent, ledit élément de marquage étant l'élément de marquage de ladite première substance de détection dudit antigène vaccinal, dans la zone de dépôt dudit antigène, et
3- on calcule le rapport desdites première et deuxième valeurs, et
4- on compare la valeur dudit rapport à celle d'un rapport de référence obtenu avec une collection de sérums de référence positifs et négatifs, permettant ainsi par comparaison de déterminer la nécessité ou non de vacciner la personne pour ledit antigène vaccinal selon la valeur du rapport desdites première et deuxième valeurs.

La présente invention fournit également une trousse de diagnostic utile pour la mise en oeuvre d'une méthode selon l'invention, **caractérisée en ce qu**'elle comprend :
- au moins un dit support solide, de préférence un unique sur support solide, sur le(s)quel(s) sont fixés au moins un dit antigène microbien et desdits antigènes de contrôle, et
- la (ou les) dite(s) substance(s) de détection et le cas échéant des réactifs utiles pour la révélation desdits éléments de marquage, ladite trousse étant telle que définie dans les revendications 24 et 25.

De préférence, une trousse selon l'invention, comprend un même dit support solide sur lequel sont fixés par adsorption physique au moins un dit antigène corpusculaire et lesdits antigènes de contrôle.

Avantageusement, dans les méthodes et trousses de diagnostic selon l'invention, on utilise comme support solide une lame de verre ou en plastique, un tube de titrage ou un puits d'une plaque de microtitrage en plastique, et plus particulièrement, comme support solide, on peut utiliser tout dispositif adapté à la manipulation de suspensions cellulaires et bactériennes et notamment des tubes, des lames de verre ou de polymères, des tubes "bijoux" ou des plaques rigides de microtitration en polyéthylène, polystyrène, chlorure.

Avant la mise en oeuvre d'une méthode selon la présente invention, il est possible de prélever le sérum par une ponction veineuse à l'aiguille ou bien par prélèvement de sang capillaire, notamment à partir du lobe de l'oreille, de la pulpe du doigt, du talon, couplé à un recueil sur un disque de papier filtre ou de préférence directement dans un tampon permettant l'élution du sérum.

Selon une autre caractéristique avantageuse de la présente invention, on réalise, après un prélèvement de sang capillaire dans un tube capillaire permettant de recueillir un volume connu de sang total, le recueil dudit échantillon dans un flacon contenant un volume connu d'un tampon d'élution. Cette modalité de recueil originale présente comme avantages sa rapidité (les recueil, élution, et dilution se faisant en une minute). Le fait que le sérum est dilué à une concentration connue, notamment de 1/20 à 1/100, est une sécurité de manipulation vis à vis du risque d'accidents d'exposition au sang tels que transmission de virus et autres pathogènes sanguins pour le préleveur.

Par ailleurs, la présente invention permet la détermination du statut vaccinal de la personne contre plusieurs simultanément contre plusieurs antigènes vaccinaux, sur un seul échantillon de sérum prélevé par ponction capillaire, dans un laps de temps inférieur à 2 heures.

Avantageusement, on recueille un volume déterminé de sang total à l'aide d'un tube capillaire dans un flacon contenant un volume déterminé d'un tampon permettant l'élution du sérum, le sérum étant alors de préférence dilué à une concentration déterminée, de préférence de 1/100 à 1/20, et l'on dépose un volume déterminé de sérum ainsi dilué sur les différentes zones de dépôt desdits antigènes de contrôle et antigènes vaccinaux sur ledit support solide.

Quelque soit le mode de recueil du sang, "prise de sang" ou "ponction capillaire", il convient dans un premier temps de séparer le sérum des globules sanguins. Dans le cas de la ponction capillaire, cette séparation est réalisée par une élution par un tampon d'élution, et c'est le produit de cette élution qui est mis en contact avec la lame.

Et avantageusement donc, la trousse comprend un flacon comportant un volume déterminé d'un tampon d'élution pour le recueil d'un volume déterminé d'un échantillon de sérum à tester.

La présente invention a également pour objet une méthode de préparation d'un support solide d'une trousse de diagnostic selon l'une des revendications 24 à 26, sur lequel sont fixés au moins un dit antigène microbien, de préférence corpusculaire, un dit premier, un dit deuxième, et de préférence un dit troisième et un dit quatrième antigènes de contrôle permettant une détection par lecture automatisée à l'aide d'une dite première et, le cas échéant, deuxième substance de détection, **caractérisée en ce que** l'on dépose de façon robotisée lesdits antigènes microbiens, de préférence corpusculaires, et antigènes de contrôle avec un robot de dépôt comprenant de préférence une seringue, lesdits antigènes étant de préférence associés à un colorant, de préférence encore un colorant fluorescent sous forme de suspension à une concentration permettant leur visualisation après dépôt à l'aide dudit colorant, et permettant ainsi de vérifier la fixation des dits antigènes sur ledit support solide.

De préférence, on dépose de façon robotisée un dit antigène microbien et, le cas échéant, un dit deuxième antigène de contrôle et, le cas échéant, un dit quatrième antigène de contrôle, sous forme de suspension de corpuscules de cellules non confluentes, virus entiers ou bactéries entières ou fractions de cellules ou bactéries.

Selon la présente invention, les inventeurs ont mis au point, après de nombreux essais infructueux, des conditions de dépôt robotisé d'antigènes microbiens ou vaccinaux corpusculaires (microbe entier inactivé ou fraction de microbe entier inactivé) en suspension dans un milieu de dépôt. En effet, actuellement, on dépose sur support solide, par les robots de dépôt, uniquement des solutions homogènes d'une ou de plusieurs molécules. Pour ce faire, la concentration de ces antigènes corpusculaires est calibrée avant dépôt par comptage des particules microbiennes inactivées par « fluorescence activated cell sorting (FACS-scann) puis déposés de façon robotisée sur un support solide. La mise au point de ces dépôts calibrés et robotisés comporte la détermination par des essais, de la concentration optimale pour chacun des antigènes testés, les concentrations infra-optimales donnant des dépôts indétectables, les concentrations supra-optimales entraînant une sédimentation des antigènes corpusculaires de forte densité et de dimensions micrométriques tels que les bactéries entières ou fractionnées en cours de dépôt et donc une variation sensible de la quantité d'antigène déposé. Enfin, les dépôts d'antigènes corpusculaires comportant de l'ADN microbien (bactérie, virus, parasites ou champignons microscopiques) sont calibrés par application d'un colorant de préférence fluorescent, notamment une molécule comme l'AMCA qui se fixe de façon non spécifique aux protéines contenues dans l'antigène ou une molécule intercalante qui se fixe de façon non spécifique à l'ADN par intercalation dans la double hélice. Cette dernière méthode est utilisée de préférence pour marquer les cellules utilisées comme témoin sur les lames. Les longueurs d'onde d'excitation et d'émission sont avantageusement choisies en fonction de celles utilisées par le fluorochrome marquant les immunoglobulines de détection. Ce marquage fluorescent, non spécifique des antigènes peut être réalisé avant leur dépôt par le robot de dépôt ou après celui-ci. Le marqueur fluorescent est avantageusement choisi pour sa stabilité à la lumière du jour.

Plus particulièrement, dans la méthode de préparation de support solide selon l'invention, les antigènes de contrôle sous forme de suspension de cellules sont calibrés à une concentration de 10⁷ à 10⁹ cellules/ml, et lesdits agents de contrôle ou antigènes microbiens sous forme de suspensions de bactéries ou fractions de bactéries à une concentration de 10⁷ à 10⁹ particules/ml et les suspensions de virus entiers à une concentration de 10⁹ à 10¹⁰ particules/ml.

Avantageusement, on dépose lesdits antigènes de contrôle et microbiens ou vaccinaux corpusculaires en mélange avec un liant protéique, stabilisant la fixation sur ledit support solide.

Plus particulièrement, ledit liant protéique est choisi parmi le mélange organique complexe formé par le jaune d'oeufs, de la gélatine, de l'albumine de sérum de bovin ou une IgG polyclonale non humaine, de préférence de chèvre.

Ces liants protéiques fonctionnent comme une colle biologique dudit antigène sur le support solide;

Les différents liants ont été testés sur lame de verre et on a déterminé les concentrations optimales de mise en oeuvre. On peut notamment utiliser l'albumine sérique bovine à une concentration finale (volume pour volume) de 1 à 5%, une suspension de jaune d'oeuf à une concentration finale de 1 à 10% et ladite IgG caprine à une concentration finale de 25 à 75%.

Les inventeurs ont remarqué au cours de différents essais réalisés, que certains antigènes liés par de l'oeuf ou de l'albumine bovine étaient lavés lors des étapes de lavage mais que les immunoglobulines humaines IgG introduites comme contrôle restaient invariablement fixées sur la lame de verre. Les inventeurs en ont déduit que les immunoglobulines de classe G se fixaient à la lame de verre d'une façon telle qu'elles supportaient les étapes de lavage et ont formé l'hypothèse que ces IgG permettraient donc de fixer également sous des conditions appropriées certains antigènes vaccinaux. Les inventeurs ont donc utiliser des IgG d'une autre espèce que l'homme, afin de ne pas interférer dans les tests sérologiques et les auteurs ont ainsi déterminé les propriétés remarquables des IgG de chèvre à titre de colle biologique permettant de fixer les antigènes particulaires, notamment les antigènes vaccinaux particulaires ou corpusculaires.

De préférence et plus particulièrement, ledit antigène vaccinal corpusculaire est déposé sur ledit support solide constitué d'une lame de verre, en mélange avec une immunoglobuline de type IgG polyclonale de chèvre.

Plus particulièrement, la méthode de préparation d'un support solide selon l'invention est **caractérisée en ce que** l'on réalise un lavage préalable du dit support solide avec une solution d'un mélange éthanol/acétone, de préférence à 50/50, puis on dépose et on stabilise la fixation des dits antigènes par adsorption physique sur ledit support solide par un traitement avec de l'alcool, de préférence méthanol ou éthanol, alcool que l'on élimine ensuite et, de préférence, on vérifie la fixation des dits antigènes par coloration, notamment par un marquage fluorescent non spécifique des protéines ou de l'ADN tel qu'explicité ci-dessus.

Cette solution de prélavage permet de nettoyer le support de toute trace de substance de détection ou autre résiduelle et, notamment, éliminer toute fluorescence, tout en conservant la faculté d'adsorption physique du support vis à vis des dits antigènes déposées ensuite.

Le traitement de stabilisation à l'alcool permet de stabiliser la fixation par adsorption physique aussi bien des protéines, telles que IgG, que des antigènes particulaires.

La détermination du lavage préalable approprié du support solide, notamment de la lame de verre a donné lieu à de nombreux essais. Ce traitement a pour objectif de nettoyer parfaitement ce support pour éliminer les artéfacts fluorescents tout en préservant la fixation ultérieure des antigènes de façon compatible avec leur mode de conservation mais, aussi, en préservant sinon l'intégrité au moins la réactivité immunologique des antigènes. Il a été montré, après de nombreuses recherches infructueuses, que le rinçage et le nettoyage des lames en phase aqueuse ne permettraient pas une fixation ultérieure des antigènes à déposer ; il en allait de même pour le rinçage à base de molécules tensio-actives comme le Tween 20. Le nettoyage avec des alcools n'était pas suffisant pour retirer la plupart des artéfacts fluorescents. C'est donc au terme de ces multiples essais qu'a été optimisé un protocole de nettoyage de support solide, notamment de la lame de verre, par un mélange éthanol 50 % - acétone 50 % puis séchage à l'air.

Toutefois, même dans ce cas, il reste avantageux de compléter la fixation par adsorption physique par un traitement de réticulation, notamment par traitement chimique avec un agent bi-fonctionnel de couplage covalent tel que le glutaraldéhyde ou des dérivés diacides activés, notamment de l'acide succinimique, connus de l'homme de l'art pour assurer des pontages covalents entre lesdits antigènes de contrôle et microbiens avec le support solide.

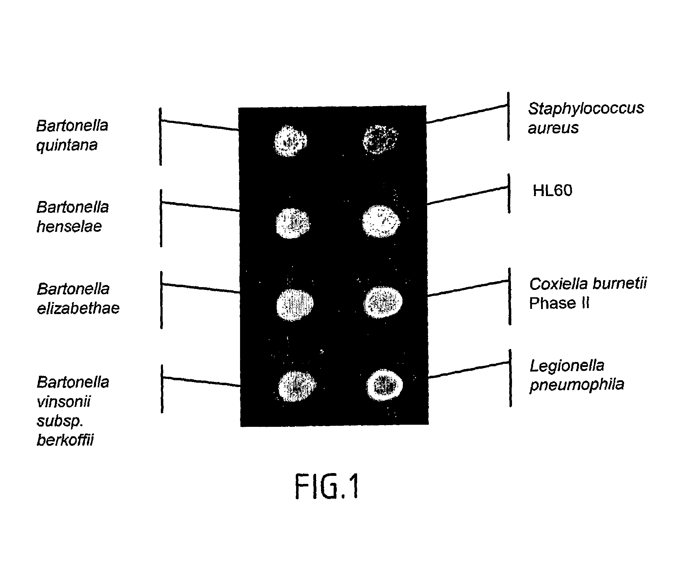

D'autres caractéristiques et avantages de la présente invention apparaîtront à la lumière des exemples qui vont suivre qui font référence aux figures 1 à 3.

La figure 1 représente une lame colorée par un colorant fluorescent, permettant de visualiser l'ensemble des 8 antigènes déposés, cellules HL60, *Staphylococcus aureus, Coxiella burnetii, Legionella pneumophila, Bartonella henselae, Bartonella quintana, Barionella vinsonii subsp. berkoffii,* et *Bartonella elizabethae* à l'exemple 3.

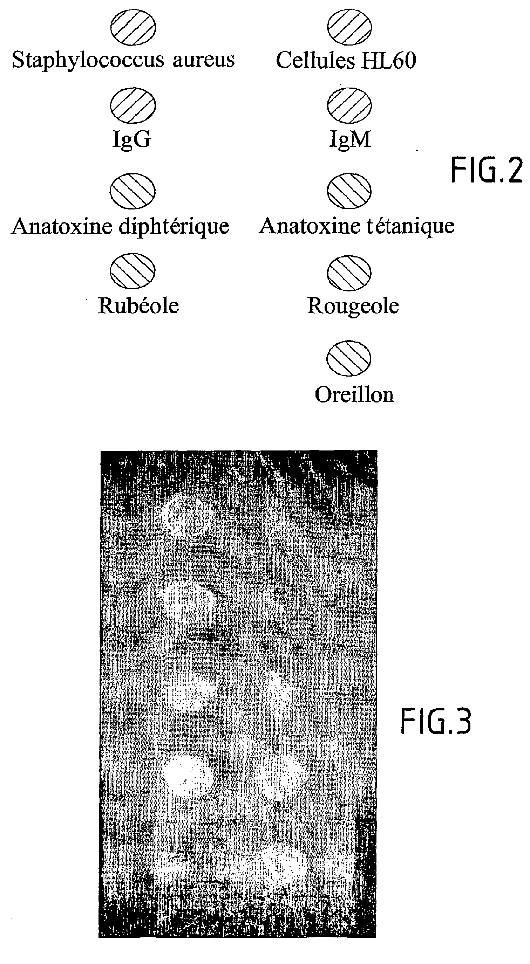

La figure 2 est un schéma indiquant le plan des dépôts d'antigènes sur une lame utilisée à l'exemple 4.

La figure 3 est l'image de la lame de l'exemple 4, après incubation avec quatre sérum, obtenus à 350 nanomètres (coloration fluorescente non spécifique avec marquage fluorescent non spécifique des protéines à l'AMCA et de l'ADN au colorant HOESCHT 332-42 conformément à l'exemple 4. Exemple 1 : Dépôt de dits deuxième et quatrième antigènes sur support solide.

Les cellules HL60 (N° ATCC CCL 240) sont des cellules fibroblastiques humaines en lignée continue utilisées pour la détection des anticorps anti-nucléaires. Après culture et production selon les protocoles habituels, on a quantifié la concentration des cellules HL 60 à l'aide d'un compteur de cellules (Microcytes® BPC / Yeast, BioDETECT AS, Olso, Norvège) et cette concentration a été rapportée à 10⁸ cellules / mL dans un tampon BPS stérile, pH= 7,4 pour obtenir une suspension de cellules non confluentes pouvant être déposées par robot de dépôt.

*Staphylococcus aureus* (n° de dépôt ATCC 29213) a été cultivé sur gélose à 5% de sang de mouton, puis récolté dans un tampon PBS stérile contenant 0,1% d'azide de sodium. L'inoculum a été mesuré à l'aide du même compteur de cellules et calibré à 10⁹ bactéries/mL qui est la concentration optimum compte tenu des contraintes d'absence de sédimentation en cours de dépôt et d'un dépôt en quantité suffisante de particules staphylococciques puis conservé par congélation à - 80°C.

On a déposé ces cellules HL60 et des bactéries *Staphylococcus aureus* à une concentration de 10⁹ CFU/mL déterminée par FACS-scan (Microcytes®) des lames en verre (Référence LLR2-45, CML, Nemours, France). Les cellules et les bactéries ont été déposées sur le support solide à l'aide d'un robot de dépôt (Arrayer 427 ™, Affymetrix, MWG Biotech SA, Courtaboeuf, France). Les dépôts ont été séchés à l'air pendant 30 minutes dans l'enceinte du "spotter", puis fixés dans un bain de méthanol à 100 % pendant 10 minutes, puis séchés à nouveau à l'air libre. L'efficacité du dépôt robotisé sur les lames, après la fixation à l'éthanol et après les bains nécessités par la réaction d'immunofluorescence indirecte suivante, a été vérifiée avec succès par la coloration par le colorant fluorescent Hoescht 333-42 qui est excité à 350 nanomètres et qui émet à 460 nanomètres (Molecular Probes). La figure 1 illustre le dépôt robotisé de 6 colonnes de 6 dépôts chacune de *Staphylococcus aureus.*

### Exemple 2 : dépôt de dits premier antigène (IgG) et troisième antigène (IgM) sur support solide

Des immunoglobulines humaines de classe G γ-spécifiques (IgG) (Serotec, Cergy Saint-Christophe, France) diluées à une concentration de 5mg/mL, et des immunoglobulines de classe M γ-spécifiques (IgM (Serotec) diluées à une concentration de 10 mg/mL pour obtenir des spots bien homogènes, ont été déposées de façon robotisée à l'aide d'un robot de dépôt (modèle 427, Arrayer, Affymetrix, Inc., CA) sur un support solide constitué d'une lame de verre (Référence LLR2-45, CML, Nemours, France).

Les dépôts ont été réalisés par transfert de la suspension d'antigène d'un puit de plaque de microtitration contenant 25 µL de suspension, sous un volume de 1 mL déposé, à 25°C et 55 % d'humidité dans l'enceinte du robot de dépôt. Ces conditions ont été contrôlées par un thermo-hygromètre. Les dépôts d'une dimension de 200 µm ont été séchés à l'air pendant 30 minutes à 37°C dans l'enceinte du robot de dépôt, puis fixés dans un bain d'éthanol à 100% pendant 10 minutes, puis séchés à nouveau à l'air libre. L'efficacité du dépôt robotisé puis de la fixation à l'éthanol après les bains nécessités par la réaction d'immunofluorescence indirecte a été vérifiée par la technique d'immunofluorescence indirecte.

10 sérums humains contenant des facteurs rhumatoïdes et 10 sérums humains ne contenant pas de facteurs rhumatoïdes (contrôles négatifs) pour l'immunodétection des facteurs rhumatoïdes, [détection qualitative par un test d'agglutination de billes de latex sensibilisées (Rhumalatex Fumouze®, Laboratoires Fumouze, Levallois-Perret France)] et ont été déposés chacun sur un dépôt d'IgG sous trois dilutions chacun, 1 :32, 1 :64 et 1 :128. Après un premier lavage, une réaction d'immunofluorescence indirecte a été réalisée en utilisant une immunoglobuline de chèvre (référence A-11013, Molecular Probes, Eugene, USA) à titre d'anticorps secondaire anti-IgM humaines, couplée à l'Alexa 488 qui est une molécule fluorescente excitée à 488 nanomètres et émettant à 540 nanomètres et en utilisant la même immunoglobuline couplé à l'Axa 594 (A-21216, Molecular Probes, Eugene, USA) qui est une molécule fluorescente excitée à 594 nanomètres et émettant à 640 nanomètres dans une deuxième expérience. La lecture de la réaction a été faite au microscope à fluorescence et a montré une détection fluorescente dans tous les sérums contenant des facteurs rhumatoïdes sous forme d'un spot très brillant pour chacune des 3 dilutions testées au niveau des dépôts d'Ig₁ et aucune fluorescence dans les sérums ne contenant pas ces facteurs.

Cet exemple illustre également qu'il est possible de réaliser un dépôt robotisé d'IgG et d'IgM humaines sur un support solide dans des conditions compatibles avec la réalisation d'une réaction d'immunofluorescence indirecte pour la détection de facteurs rhumatoïdes. Egalement, cet exemple illustre qu'il est possible de réaliser la détection de facteurs rhumatoïdes par une technique d'immunofluorescence indirecte.

Trois concentrations d'IgM ont été déposées de haut en bas : 10 mg/ml, 1 mg/ml et 0,2 mg/ml, six dépôts ont été réalisés par concentration. La concentration de 10mg/ml permet d'obtenir les spots les plus homogènes.

### Exemple 3 : Un diagnostic sérologique d'infection à Coxiella burnetii a été réalisé selon le procédé décrit dans la présente invention

Pour la réalisation des différents tests sérologiques, 8 antigènes ont été utilisés : Cellules HL 60 (à titre de dit deuxième antigène), *Staphylococcus aureus* (à titre de dit quatrième antigène), IgG humaines (à titre de dit premier antigène) et *Coxiella burnetii* Nine Mile, phase II (N° A.T.C.C. VR 616), *Legionella pneumophila, Bartonella benselae, Bartonella quintana, Bartonella vinsonii* subsp. *berkoffii,* et *Bartonella elizabethae* (à titre de dit antigènes microbiens).

Après culture et production selon les protocoles habituels, on a quantifié la concentration des cellules HL 60 à l'aide d'un compteur de cellules (Microcytes® BPC / Yeast, BioDETECT AS, Olso, Norvège) et cette concentration a été rapportée à 10⁷ cellules / mL dans un tampon BPS stérile, pH= 7,4 pour obtenir une suspension de cellules non confluentes déposable par robot de dépôt. Ces cellules ont été conservées dans de l'éthanol 100 % à + 4°C avant leur dépôt robotisé.

*Staphylococcus aureus* a été cultivé sur gélose à 5 % de sang de mouton puis récolté dans un tampon PBS stérile contenant 0,1 % d'acide de sodium. L'inoculum a été mesuré à l'aide du même compteur de cellules et calibré à 10⁹ bactéries/mL puis conservé par congélation à -80°C.

Des immunoglobulines humaines de classe G (IgG) (Serotec, Cergy Saint-Christophe, France) ont été diluées extemporanément à une concentration de 5mg/Ml.

Enfin, la bactérie *Coxiella burnetii* Nine Mile phase II a été cultivée sur support cellulaire et purifiée selon les protocoles habituels et quantifiée par le compteur Microcyte® afin de standardiser sa concentration à 10⁹ bactéries/mL qui est la concentration optimum compte tenu des contraintes d'absence de sédimentation en cours de dépôt et d'un dépôt en quantité suffisante des particules bactériennes. Cet antigène a été conservé à -80°C dans un tampon PBS, 0,1 % acide de sodium.

Ces 4 antigènes ont été déposés sur le support solide (Référence LLR2-45, CML, Nemours, France) à l'aide d'un robot de dépôt (Arrayer 427 ™, Affymetrix, MWG Biotech SA, Courtaboeuf, France).

Le support solide consistait en une lame de verre préalablement nettoyée par un mélange éthanol 50 % - acétone 50 % puis séchée à l'air ambiant. Les dépôts ont été séchés à l'air pendant 30 minutes dans l'enceinte du robot de dépôt, puis fixés dans un bain de méthanol à 100 % pendant 10 minutes, puis séchés à nouveau à l'air libre. Les dépôts ont été réalisés par transfert de la suspension d'antigène d'un puit de plaque de microtitration contenant 25 µL de suspension, sous un volume de 1 mL déposé, à 25°C et 55 % d'humidité dans l'enceinte du robot de dépôt. Ces conditions ont été contrôlées par un thermo-hygromètre. Les dépôts d'une dimension de 200 µm ont été séchés à l'air pendant 30 minutes à 37°C dans l'enceinte du robot de dépôt, puis fixés dans un bain de méthanol à 100% pendant 10 minutes, puis séchés à nouveau à l'air libre.

Pour réaliser le test sérologique, 5 sérums humains contenant des facteurs rhumatoïdes, 5 sérums humains contenant des anticorps anti-nucléaires, 10 sérums humains présentant à la fois des anticorps de type IgG anti-*Coxiella burnetii* phase II à un titre ≥ 1 :200 et des anticorps de type IgM anti-*Coxiella burnetti* phase II à un titre ≥ 1 :50 et 10 sérums humains témoins négatifs ne contenant aucun de ces 3 types d'anticorps ont été utilisés et ont été déposés chacun sur un dépôt d'IgG sous trois dilutions chacun, 1 :32, 1 :64 et 1 :128.

Après un premier lavage, des réactions d'immunofluorescence indirecte ont été réalisées utilisant :
- un premier anticorps secondaire constitué d'une immunoglobuline de chèvre anti IgM humaines, couplé à l'Alexa 488 (A-11013, Molecular Probes, Eugene, USA) qui est une molécule fluorescente excitée à 488 nanomètres et émettant à 540 nanomètres, et
- un second anticorps secondaire constitué d'une immunoglobuline de chèvre anti IgG humaines couplée à l'élément de marquage Alexa 594 (A-21216, Molecular Probes, Eugene, USA) qui est une molécule fluorescente excitée à 594 nanomètres et émettant à 640 nanomètres dans une deuxième expérience.

Ces réactions ont été réalisées à température ambiante, dans une enceinte humide, comportant 15 minutes d'incubation avec le sérum à tester, suivies d'un lavage au tampon PBS stérile puis d'une incubation de 5 minutes avec les anticorps conjugués, avant un deuxième rinçage. La lecture des réactions a été faite au microscope à fluorescence et a donné lieu à des enregistrements numérisés des spots. Au cours de ce test :
- les 30 sera ou sérums humains ont "allumé" le dépôt de *S. aureus,* c'est-à-dire réagi avec ledit quatrième antigène et ladite immunoglobuline de chèvre anti IgG couplée à l'Alexa 594,
- seuls les sérums contenant de facteurs rhumatoïdes ont "allumé" le dépôt d'IgG lors de la lecture du marqueur Alexa 488 de ladite immunoglobuline anti IgM,
- seuls les sérums contenant des anticorps anti-nucléaires ont "allumé" le dépôt de cellules HL60 lors de la lecture avec ladite seconde substance de détection anti IgG couplée à l'Alexa 594, et
- seuls les sérums contenant des anticorps contre *C. burnetii* ont "allumé" le dépôt de *C. burnetii* lors de la lecture avec l'une quelconque des deux substances de détection anti IgM et anti IgG,
- toutes les réactions utilisant les anticorps secondaires conjugués anti IgG ont "allumé" le dépôt IgG.

Les titres observés d'anticorps anti-*C. burnetii* étaient concordants avec ceux retrouvés par la méthode référencée (Micro-immunofluorescence indirecte) [Dupont HT, Thirion X, Raoult D.Q fever serology: cutoff determination for microimmunofluorescence. Clin Diagn Lab Immunol. 1994;1:189-96].

La figure 1 représente une lame colorée par le colorant fluorescent Hoescht 333-42 qui est excité à 350 nanomètres et qui émet à 460 nanomètres (Molecular Probes), permettant de visualiser l'ensemble de 8 antigènes déposés, cellules HL60, *Staphylococcus aureus, Coxiella burnetii, Legionella pneumophila, Bartonella henselae, Bartonella quintana, Bartonella vinsonii* subsp. *berkoffii,* et *Bartonella elizabethae.* Ces différents antigènes ont été déposés dans les mêmes conditions que *Coxiella,* décrites ci-dessus.

### Exemple 4 : Détermination du statut vaccinal de personnes.

On a mis au point une lame de verre pour la détermination du statut vaccinal des personnes comportant un total de 8 dépôts d'antigènes comportant 3 dépôts de contrôles et 5 dépôts d'antigènes vaccinaux sur une même lame, répartis en 2 colonnes. Les modalités de dépôt et d'utilisation des 3 dépôts contrôles comportant Staphylococcus aureus, IgG et cellules HL-60 ont été exposes dans les exemples 1 et 2 ci-dessus.

Les dépôts d'antigènes vaccinaux ont été réalisés sous forme de suspension virale de virus entiers vivants inactivés déposés à une concentration de 10⁹ ou 10¹⁰ particules/ml en mélange avec une IgG caprine à une concentration de 25 à 75% (volume pour volume) et suivant les modalités suivantes :
(1) antigène rubéole : il s'agit de la souche hpv-77 (microbix Biosystems, Inc., Toronto, Ontario, Canada) fournie sous forme d'une suspension virale inactivée à concentration protéique de 0, 51 mg/ml. Cet antigène a été concentré 10 fois par centrifugation avant son dépôt sous un volume de 45 µL d'antigène et 5 µL d'immunoglobulines IgG de chèvre titrant à 10 mg/ml (référence I5256, Sigma, Saint-Quentin Fallavier, France), colorées par 2 µL d'amino methyl coumarin acetyl (AMCA) (Molecular Probes, Interchim, Montluçon, France).
   L'AMCA se fixe de façon non spécifique aux protéines de cet antigène et permet donc de vérifier le dépôt de l'antigène sur le support solide par adsorption physique. Il est excité à une même longueur d'onde de 350 nm que le colorant Hoescht 333-42.
(2) antigène rougeole : il s'agit de la souche Edmonston (Microbix Biosystems, Inc.) fournie sous forme d'une suspension de particules virales inactivées à une concentration protéique de 1,95 mg/ml qui a été concentrée 5 fois par centrifugation avant son dépôt sous un volume de 45 µL d'antigène et 5 µL d'immunoglobuline IgG de chèvre colorée à l'AMCA.
(3) Antigène oreillons : il s'agit de la souche Enders (société microbix-biosystem incorporated) titrant à une concentration protéique de 1,8 µg/ml et déposée sous un volume de 45 µL d'antigène et 5 µL d'immunoglobiline IgG de chèvre marqués à l'AMCA.
(4) anatoxine diphtérique (référence FA 150934, Aventis Pasteur Vaccins) titrant à une concentration protéïque de 18,9 mg/ml et concentrée 10 fois avant son dépôt sous un volume de 45 µL d'antigène et 5 µL d'immunoglobulines IgG de chèvre marquées à l'AMCA,
(5) anatoxine tétanique (référence J001, Aventis Pasteur vaccins) titrant à une concentration de 80 UI/mL et déposée pure sous un volume de 45 µL d'antigène et 5 µL d'immunoglobuline IgG de chèvre marquées à l'AMCA.

Les dépôts d'antigènes ont été réalisés sous un volume de 1 nl par un "spotter" de marque Affymetrix®, de modèle Arrayer 427. Après séchage, les lames ainsi préparées ont servi de support à une réaction d'immunofluorescence indirecte pour la détection des IgG spécifiques des antigènes vaccinaux dans le sérum des personnes selon le protocole suivant
- dans une première étape, 5 µL de sérum prélevé par ponction veineuse ou par ponction capillaire, sont mis en contact et incubés avec la lame au niveau des différents antigènes vaccinaux et antigènes de contrôles, par un automate d'incubation.
- dans une deuxième étape, l'automate rince la lame afin d'éliminer le sérum testé et réalise une incubation avec l'anticorps de détection anti-IgG humaines conjugué à la fluorescéine qui est excitée à une longueur d'onde de 488 nm (référence Star 106 F, Serotec, France),
- dans une troisième étape, l'automate rince la lame afin d'éliminer l'anticorps de détection conjugué, et sèche la lame,
- dans une quatrième étape, la lame ainsi traitée est retirée de l'automate d'incubation et placée dans la chambre de lecture d'un lecteur automatique de fluorescence. Ce lecteur réalise successivement deux lectures, une à 350 nm qui est la longueur d'onde d'émission du colorant AMCA puis une deuxième lecture à 488 nm qui est la longueur d'onde d'émission de la fluorescéine de l'anticorps de détection,
- dans une cinquième étape, ces données sont transmises à un logiciel afin d'être converties en intensité de fluorescence à 350 nm et à 488 nm pour chacun des dépôts,
- dans un sixième étape, le logiciel analyse les niveaux de fluorescence des contrôles et vérifie successivement
   . la présence de fluorescence pour le dépôt Staphylococcus aureus pour vérifier la présence du sérum à tester ;
   . la présence de fluorescence du dépôt d'IgG pour vérifier la qualité de la réaction impliquant l'anticorps de détection conjugué ;
   . l'absence de fluorescence du dépôt de cellules HL60 pour vérifier la présence ou l'absence d'anticorps anti-nucléaires dans le sérum à tester.
- dans une septième étape, le logiciel calcule le ratio de fluorescence 488/350 pour chacun des dépôts d'antigènes vaccinaux et compare pour chaque dépôt d'antigène vaccinal cette valeur à une courbe de ratio préalablement établie pour chaque antigène vaccinal à l'aide de sérums témoins positifs possédant des anticorps spécifiques à une concentration connue par une méthode de référence et de sérums négatifs ne possédant pas d'anticorps spécifiques détectables par une méthode de référence,

La fluorescence à 350 nn qui résulte de l'excitation de marqueurs non-spécifiques qui se fixent de façon non-spécifique soit à l'ADN soit aux protéines des antigènes vaccinaux. La quantité de fluorescence émise par les dépôts d'antigènes à 350 nn est proportionnelle à la quantité d'antigènes effectivement présents dans le dépôt, de sorte que cette fluorescence à 350 nn est une mesure dépendant de la quantité d'antigènes présente dans le dépôt. La quantité de fluorescence à 350 nn est utilisée par le logiciel
. d'une part pour repérer la position topographique des dépôts d'antigènes sur la lame et
. d'autre part pour en quantifier la surface exacte et donc les contours afin de ne pas incorporer des artéfacts de fluorescence qui seraient situés en dehors de ces spots, et
. enfin, pour pondérer la fluorescence à 488 nn. Cette deuxième longueur d'onde de 488 nn, résulte de l'excitation du marqueur de l'Immunoglobuline G de détection. La quantité de fluorescence à 488 nn est en relation avec la quantité d'Immunoglobuline G spécifique présente dans le spot du sérum du patient à tester. La fluorescence à 488 nn (et donc la fixation des Immunoglobulines G) dépend de la quantité d'antigènes qui ont été déposés. En effet, si l'on dépose très peu d'antigène, la quantité d'Immunoglobuline spécifique fixée va être faible et donc le niveau de fluorescence à 488 va être faible. C'est pourquoi la quantité de fluorescence à 488 nn est pondérée pour chacun des spots d'antigènes vaccinaux par la quantité de fluorescence à 350 nn et le ratio des fluorescences exprimant la quantité d'IgG spécifiques présente dans le sérum testé.

- dans une dernière étape, le logiciel interprète par comparaison des ratios de fluorescence 488/350 mesurés avec les sérums testés avec ceux obtenus avec une collection de sérums de références positifs et négatifs pour chacun qui ont permis d'établir les courbes de référence, l'ensemble de ces données pour indiquer la liste des antigènes vaccinaux contre lesquels le sérum testé possède des anticorps spécifiques, et donc le statut vaccinal de la personne.
- Ainsi, dans cet exemple on a testé quatre sérums prélevés chez quatre patients différents pour rechercher la présence d'anticorps de classe IgG contre les antigènes vaccinaux de la rougeole, de la rubéole, des oreillons, de la diphtérie et du tétanos. La valeur seuil du ratio de fluorescence a été déterminée pour chacun de ces 5 antigènes vaccinaux.
- Les tableaux 1A, 1B, 1C et 1D montrent les ratios de fluorescence pour chacun des 4 sérums testés, respectivement.

La lecture des tableaux 1A à 1C indique que :
- le sérum n°1 est :
   . positif pour la présence d'anticorps anti-anatoxine diphtérique (ratio de fluorescence > 1, valeur seuil),
   . positif pour la présence d'anticorps anti-anatoxine tétanique (ratio de fluorescence > 0,05, valeur seuil),
   . négatif pour la présence d'anticorps anti-rubéole (ratio de fluorescence < 0,1, valeur seuil),
   . positif pour la présence d'anticorps anti-rougeole (ratio de fluorescence > 0,1, valeur seuil), et
   . positif pour la présence d'anticorps anti-oreillons (ratio de fluorescence > 0,11, valeur seuil) ;
- le sérum n°2 est :
   . positif pour la présence d'anticorps anti-anatoxine diphtérique (ratio de fluorescence > 1, valeur seuil),
   . positif pour la présence d'anticorps anti-anatoxine tétanique (ratio de fluorescence > 0,05, valeur seuil),
   positif pour la présence d'anticorps anti-rubéole (ratio de fluorescence > 0,1, valeur seuil),
   . positif pour la présence d'anticorps anti-rougeole (ratio de fluorescence > 0,1, valeur seuil), et
   . positif pour la présence d'anticorps anti-oreillons (ratio de fluorescence > 0,11, valeur seuil) ;
- le sérum n°3 est :
   . négatif pour la présence d'anticorps anti-anatoxine diphtérique (ratio de fluorescence < 1, valeur seuil),
   . négatif pour la présence d'anticorps anti-anatoxine tétanique (ratio de fluorescence < 0,05, valeur seuil),
   . négatif pour la présence d'anticorps anti-rubéole (ratio de fluorescence < 0,1, valeur seuil),
   . positif pour la présence d'anticorps anti-rougeole (ratio de fluorescence > 0,1, valeur seuil), et
   . négatif pour la présence d'anticorps anti-oreillons (ratio de fluorescence < 0,11, valeur seuil) ;
- le sérum n°4 est :
   . négatif pour la présence d'anticorps anti-anatoxine diphtérique (ratio de fluorescence < 1, valeur seuil),
   . négatif pour la présence d'anticorps anti-anatoxine tétanique (ratio de fluorescence < 0,05, valeur seuil),
   .positif pour la présence d'anticorps anti-rubéole (ratio de fluorescence > 0,1, valeur seuil),
   . négatif pour la présence d'anticorps anti-rougeole (ratio de fluorescence < 0,1, valeur seuil), et
   . négatif pour la présence d'anticorps anti-oreillons (ratio de fluorescence égale à 0,11, valeur seuil).
- La figure 2 est un schéma indiquant le plan des dépôts d'antigènes sur la lame vaccin.
- La figure 3 est l'image de cette lame après incubation avec les 4 sérums cités ci-dessus, obtenue à 350 nm (coloration fluorescente non spécifique après marquage fluorescent non-spécifique des protéines à l'AMCA et de l'ADN au colorant Hoescht 332-42). Cette image permet de contrôler la présence de chacun des dépôts d'antigène de contrôle et vaccinaux.

Sur les figures 2 et 3, apparaît un spot (zone de dépôt) IgM qui n'est pas utile dans les tests réalisés.

**Tableau 1A**

| Sérum N° 1 | | | | | | |
|---|---|---|---|---|---|---|
| Numéro | Abscisse | Ordonnée | Surface | Fluorescence 350 | Ratio F350/F488 | Fluorescence 488 |
| SA | 240 | 127 | 1228 | 44238 | 1,274 | 56359 |
| IgG | 238 | 195 | 1198 | 54695 | 0,902 | 49335 |
| Diphtérie | 238 | 263 | 1203 | 44069 | 1,691 | 74521 |
| Rubéole | 235 | 332 | 1117 | 56298 | 0,075 | 4222 |
| HL | 309 | 123 | 452 | 9652 | 0,458 | 4421 |
| IgM | 306 | 197 | 918 | 18369 | 0,183 | 3362 |
| Tétanos | 304 | 266 | 842 | 26512 | 0,078 | 2068 |
| Rougeole | 303 | 334 | 1104 | 37075 | 0,13 | 4820 |
| Oreillon | 302 | 401 | 1038 | 29978 | 0,118 | 3537 |

**Tableau 1B**

| Sérum N° 2 | | | | | | |
|---|---|---|---|---|---|---|
| Numéro | Abscisse | Ordonnée | Surface | Fluorescence 350 | Ratio F488/ F350 | Fluorescence 488 |
| SA | 249 | 109 | 1199 | 41653 | 1,839 | 76600 |
| IgG | 250 | 177 | 1255 | 49420 | 1,263 | 62417 |
| Diphtérie | 251 | 247 | 1176 | 39874 | 2,350 | 93704 |
| Rubéole | 251 | 313 | 1173 | 50395 | 0,108 | 5443 |
| HL | 316 | 106 | 537 | 10853 | 0,627 | 6805 |
| IgM | 318 | 176 | 895 | 15917 | 0,249 | 3963 |
| Tétanos | 321 | 244 | 834 | 21013 | 0,357 | 7502 |
| Rougeole | 319 | 314 | 1164 | 39486 | 0,118 | 4659 |
| Oreillon | 320 | 382 | 1079 | 31342 | 0,120 | 3761 |

**Tableau 1C**

| Sérum N° 3 | | | | | | |
|---|---|---|---|---|---|---|
| Numéro | Abscisse | Ordonnée | Surface | Fluorescence 350 | Ratio F488/F350 | Fluorescence 488 |
| SA | 210 | 85 | 1175 | 34462 | 1,981 | 68269 |
| IgG | 210 | 153 | 1293 | 47678 | 1,561 | 74425 |
| Diphtérie | 210 | 220 | 1255 | 54523 | 0,342 | 18647 |
| Rubéole | 209 | 288 | 1172 | 49101 | 0,073 | 3584 |
| HL | 278 | 78 | 474 | 9180 | 0,740 | 6793 |
| IgM | 278 | 151 | 943 | 15225 | 0,335 | 5100 |
| Tétanos | 278 | 220 | 848 | 24553 | 0,046 | 1129 |
| Rougeole | 278 | 288 | 1178 | 34879 | 0,153 | 5336 |
| Oreillon | 278 | 357 | 1123 | 26949 | 0,095 | 2560 |

**Tableau 1D**

| Sérum N° 4 | | | | | | |
|---|---|---|---|---|---|---|
| Numéro | Abscisse | Ordonnée | Surface | Fluorescence 350 | Ratio F488/F350 | Fluorescence 488 |
| SA | 240 | 110 | 1202 | 40889 | 1,093 | 44692 |
| IgG | 240 | 177 | 1269 | 53557 | 1,123 | 60145 |
| Diphtérie | 242 | 246 | 1253 | 56284 | 0,316 | 17786 |
| Rubéole | 241 | 314 | 1106 | 43970 | 0,107 | 4705 |
| HL | 308 | 106 | 533 | 9932 | 0,797 | 7916 |
| IgM | 308 | 178 | 926 | 17540 | 0,309 | 5420 |
| Tétanos | 309 | 244 | 838 | 29892 | 0,049 | 1465 |
| Rougeole | 309 | 314 | 1174 | 41689 | 0,093 | 3877 |
| Oreillon | 310 | 382 | 1044 | 29666 | 0,110 | 3263 |

## Revendications

1. Méthode de diagnostic sérologique in vitro d'agent microbien par immunodétection dans laquelle on détecte la présence et, de préférence, on dose la quantité d'immunoglobulines de patient des deux classes M et G ou seulement de la classe G spécifiques d'un antigène microbien caractéristique dudit agent microbien, dans un échantillon de sérum du patient à tester, par détection et quantification d'un complexe de réaction immunologique entre ledit antigène microbien à détecter et une dite immunoglobuline spécifique de classe G pour le dosage d'IgG, ou de classe M pour le dosage d'IgM, à l'aide d'une première substance de détection constituée par un anticorps ne réagissant qu'avec une dite immunoglobuline de l'espèce du patient de classe G pour le dosage d'IgG, ou une seconde substance de détection constituée d'un anticorps ne réagissant qu'avec une dite immunoglobuline de l'espèce du patient de classe M pour le dosage d'IgM, **caractérisée en ce que** :
1/- on réalise les étapes dans lesquelles :
■ on met en contact ledit échantillon de sérum à tester puis lesdites première et seconde substances de détection ou seulement ladite première substance de détection avec au moins un support solide sur lequel ont été fixés les antigènes suivants :
- un premier antigène de contrôle constitué par une immunoglobuline non spécifique de classe G de l'espèce du patient, et
- un deuxième antigène de contrôle comprenant des complexes ADN/histones, et
- le cas échéant, un troisième antigène de contrôle constitué par une immunoglobuline non spécifique de classe M de l'espèce du patient; la présence dudit troisième antigène de contrôle étant nécessaire en cas de dosage d'IgM, et
- au moins un dit antigène microbien, et
■ on réalise une série de contrôles comprenant:
a- le contrôle de la réactivité de ladite première substance de détection en vérifiant si ledit premier antigène de contrôle réagit avec ladite première substance de détection, et, en cas de dosage d'IgM, le contrôle de la présence de facteurs rhumatoïdes dans ledit échantillon de sérum en vérifiant si le premier antigène de contrôle réagit avec ledit échantillon de sérum et ladite seconde substance de détection,
b- le contrôle de la présence d'anticorps antinucléaires dans ledit échantillon de sérum à tester en vérifiant si ledit deuxième antigène de contrôle réagit avec ledit échantillon de sérum et la première substance de détection,
c- le contrôle de la réactivité de ladite seconde substance de détection en vérifiant si ledit troisième antigène de contrôle réagit avec ladite seconde substance de détection, en cas de dosage d'IgM, et
d- le contrôle de la présence d'un sérum de l'espèce du patient dans l'échantillon à tester, et
2/- on ne prend en compte le résultat d'une réaction entre ledit antigène microbien, ledit échantillon de sérum et une dite substance de détection que si le contrôle de la présence d'un sérum de l'espèce du patient est positif et si les conditions cumulatives suivantes sont réunies:
a- ledit premier antigène de contrôle réagit avec ladite première substance de détection,
b- ledit deuxième antigène de contrôle ne réagit pas, et
c- le cas échéant, ledit troisième antigène de contrôle réagit avec ladite seconde substance de détection, en cas de dosage d'IgM.

2. Méthode selon la revendication 1, **caractérisée en ce que** l'on utilise un unique support solide mis en contact avec le cas échéant simultanément desdites première et seconde substances de détection comprenant un premier et respectivement un second élément de marquage, le second élément de marquage émettant un signal différent du premier élément de marquage, lesdites première et seconde substances de détection comprenant un premier et respectivement un second anticorps ne réagissant qu'avec une dite immunoglobuline de l'espèce du patient de classe G et respectivement de classe M.

3. Méthode selon la revendication 1 ou 2, **caractérisée en ce que** l'on contrôle que ledit échantillon testé contient bien un sérum de l'espèce du patient en détectant si des immunoglobulines de l'espèce du patient réagissent avec un quatrième antigène de contrôle contenant la protéine A d'une bactérie *Staphylococcus aureus,* de préférence ledit quatrième antigène étant une bactérie *Staphylococcus* entière, en mettant en contact ledit échantillon avec un support solide sur lequel est fixé un dit quatrième antigène de contrôle, en présence de ladite première substance de détection qui est un anticorps anti-immunoglobuline de l'espèce du patient ne réagissant pas avec ledit quatrième antigène de contrôle, le contrôle de la présence d'un sérum étant positif si ledit quatrième antigène réagit avec ledit échantillon de sérum et ladite première substance de détection.

4. Méthode selon l'une des revendications 1 à 3, **caractérisée en ce que** ladite première substance de détection, est une immunoglobuline animale, de préférence une immunoglobuline de chèvre ou de poulet.

5. Méthode selon l'une des revendications 1 à 4, **caractérisée en ce que,** le cas échéant, les deux dites première et seconde substances de détection sont des immunoglobulines de chèvre ou de poulet, respectivement anti- IgG et anti- IgM.

6. Méthode selon l'une des revendications 1 à 5, **caractérisée en ce que** ledit deuxième antigène de contrôle est constitué de cellules de fibroblastes humains.

7. Méthode selon l'une des revendications 1 à 6, **caractérisée en ce que** lesdits antigènes de contrôle et antigènes microbiens sont fixés sur support solide par adsorption physique.

8. Méthode selon l'une des revendications 1 à 7, **caractérisée en ce que** ledit antigène microbien est un antigène corpusculaire constitué de microbe entier inactivé ou une fraction de microbe.

9. Méthode de diagnostic sérologique selon l'une des revendication 1 à 8, **caractérisée en ce que** ledit agent microbien est choisi parmi des micro-organismes comprenant une bactérie, un virus, un parasite ou un champignon.

10. Méthode de diagnostic sérologique selon la revendication 9, **caractérisée en ce que** ledit antigène microbien est une bactérie intracellulaire ou un virus.

11. Méthode de diagnostic sérologique selon la revendication 9 ou 10, **caractérisée en ce que** ledit antigène microbien est choisi parmi les bactéries du genre *Rickettsia, Coxiella, Bartonella, Tropheryma, Ehrlichia, Chlamydia, Mycoplasma, Treponema, Borrelia,* et *Leptospira.*

12. Méthode de diagnostic sérologique selon la revendication 11, **caractérisée en ce que** ledit antigène microbien est une bactérie responsable d'une endocardite.

13. Méthode de diagnostic sérologique selon la revendication 10, **caractérisée en ce que** ledit antigène microbien est un antigène viral choisi parmi les virus H.I.V., C.M.V. Epstein-Barr, Rougeole, Rubéole, virus des hépatites A et B.

14. Méthode selon l'une des revendications 1 à 13, **caractérisée en ce que :**
- on détecte et on dose la quantité des immunoglobulines de patients des deux classes M et G spécifiques d'un antigène microbien,
- au moins un dit antigène microbien et lesdits premier, deuxième et troisième et quatrième antigènes de contrôle sont fixés sur un même support solide et
- on utilise pour la détection des différents dits antigènes microbiens, les mêmes dites première et seconde substances de détection avec des éléments de marquage différents, lesdites première et deuxième substances de détection étant des immunoglobulines animales ne réagissant pas avec ledit quatrième antigène.

15. Méthode selon la revendication 1 à 14, **caractérisée en ce que** l'on utilise comme support solide une lame de verre ou en plastique, un tube de titrage ou un puits d'une plaque de microtitrage en plastique.

16. Méthode selon l'une des revendications 1 à 15, **caractérisée en ce que** l'on détecte et, le cas échéant, on quantifie la dose de dite immunoglobuline de l'espèce du patient spécifique du dit antigène microbien dans l'échantillon à tester, et les réactions immunologiques entre lesdits antigènes de contrôle et lesdites substances de détection, par lecture automatisée à l'aide d'un appareil de lecture d'un signal fluorescent d'une substance fluorescente correspondant aux éléments de marquage desdites substances de détection.

17. Méthode selon l'une des revendications 1 à 16, **caractérisée en ce que** ledit antigène microbien est un antigène vaccinal et ladite immunoglobuline spécifique du dit agent vaccinal à détecter est une immunoglobuline de classe G.

18. Méthode selon la revendication 17, **caractérisée en ce que** l'on détermine le statut vaccinal d'un individu par détection de préférence quantification des anticorps sériques IgG spécifiques d'antigènes vaccinaux d'une pluralité d'agents pathogènes du type bactéries, virus, champignons ou parasites, en réalisant la détection, et de préférence la quantification, d'un complexe de réactions immunologiques entre chaque dit antigène vaccinal et respectivement chaque dit anticorps spécifique dudit antigène vaccinal, éventuellement présent dans un échantillon de sérum humain à tester, comprenant:
1- la mise en contact d'un seul et même dit échantillon de sérum à tester, puis d'au moins une dite première substance de détection réagissant avec au moins un dit anticorps spécifique et ne réagissant pas avec l'un quelconque desdits antigènes vaccinaux, avec un même support solide sur lequel est fixé une pluralité de dits antigènes vaccinaux correspondant à une pluralité d'agents pathogènes, et lesdits premier, deuxième et de préférence quatrième antigènes de contrôle, et
2- au moins un dit contrôle de la réactivité de ladite première substance de détection à l'aide d'un dit premier antigène de contrôle et un dit contrôle de la présence d'anticorps antinucléaires à l'aide d'au moins un dit deuxième antigène de contrôle, et le contrôle de la présence d'un sérum humain dans ledit échantillon à tester.

19. Méthode selon la revendication 18, **caractérisée en ce qu'**on détecte desdits anticorps spécifiques du type immunoglobulines IgG et on met en oeuvre une dite première substance de détection qui est une immunoglobuline anti-IgG, constituée d'une immunoglobuline de chèvre ou de poulet.

20. Méthode selon l'une des revendications 18 ou 19, **caractérisée en ce que** lesdits antigènes vaccinaux sont des antigènes des agents pathogènes choisis parmi les virus des oreillons, de la rubéole, de la rougeole, de la varicelle, de la poliomyélite, de la fièvre jaune, de l'encéphalite à tique, de l'hépatite A, de l'hépatite B, et les bactéries de la coqueluche *Bordetella pertussis*, du tétanos et de la diphtérie.

21. Méthode selon l'une des revendications 18 à 20, **caractérisée en ce qu'**on détermine si la concentration de dit anticorps spécifiques atteint un seuil à partir duquel ledit anticorps spécifique a une action protectrice protégeant contre la maladie déterminée par le pathogène.

22. Méthode selon l'une des revendications 18 à 21, **caractérisée en ce que** l'on recueille un volume déterminé de sang total à l'aide d'un tube capillaire dans un flacon contenant un volume déterminé d'un tampon permettant l'élution du sérum, le sérum étant alors dilué à une concentration déterminée, de préférence de 1/100 à 1/20.

23. Méthode selon l'une des revendications 18 à 22, **caractérisée en ce que,** pour chaque détection et, le cas échéant, quantification d'un dit antigène vaccinal, on réalise les mesures suivantes :
1- une première mesure d'une première valeur représentative de la quantité d'un troisième élément de marquage, ladite première valeur étant la valeur de l'intensité d'un signal émis par ledit troisième élément de marquage fluorescent, ledit troisième élément de marquage se fixant de manière non spécifique sur toute protéine dans la zone de dépôt dudit antigène vaccinal, et
2- une deuxième mesure d'une deuxième valeur représentative de la quantité d'un premier élément de marquage émettant un signal différent dudit troisième élément de marquage, ladite deuxième valeur étant la valeur de l'intensité du signal émis par ce premier élément de marquage fluorescent à une longueur d'onde d'excitation différente de celle dudit troisième élément de marquage fluorescent, ledit premier élément de marquage étant l'élément de élément de marquage de ladite première substance de détection dudit antigène vaccinal, dans la zone de dépôt dudit antigène, et
3- on calcule le rapport desdites première et deuxième valeurs, et
4- on compare la valeur dudit rapport à celle d'un rapport de référence obtenu avec une collection de sérums de référence positifs et négatifs, permettant ainsi par comparaison de déterminer la nécessité ou non de vacciner la personne pour ledit antigène vaccinal selon la valeur du rapport desdites première et deuxième valeurs.

24. Trousse de diagnostic utile pour la mise en oeuvre d'une méthode selon l'une des revendications 1 à 23, dans laquelle on réalise le dosage d'IgG, **caractérisée en ce qu'**elle comprend :
a- au moins un support solide sur lequel sont fixés au moins :
- un dit antigène microbien tel que défini dans l'une des revendications 1 et 8 à 13 et 17, 18 et 20, et
- desdits premier et deuxième antigènes de contrôle tels que définis dans l'une des revendications 1 et 6, et
- de préférence un dit quatrième antigène de contrôle tel que défini dans la revendication 3, et
b- une première substance de détection telle que définie dans l'une des revendications 1, 2, 4, 5 et 14.

25. Trousse selon la revendication 24, utile pour la mise en oeuvre d'une méthode selon l'une des revendications 1 à 23, dans laquelle on réalise en outre le dosage d'IgM, **caractérisée en ce qu'**elle comprend en outre :
a- un dit support solide sur lequel est, en outre, fixé un dit troisième antigène de contrôle tel que défini dans la revendication 1, et
b- une dite seconde substance de détection telle que définie dans l'une des revendications 1, 2, 5 et 14.

26. Trousse selon la revendication 24 ou 25, **caractérisée en ce qu'**elle comprend un même dit support solide sur lequel sont fixés par adsorption physique au moins un dit antigène corpusculaire et les dits antigènes de contrôle.

27. Méthode de préparation d'un support solide d'une trousse de diagnostic selon l'une des revendications 24 à 26, sur lequel sont fixés au moins un dit antigène microbien et desdits premier et deuxième antigènes de contrôle et, de préférence, desdits troisième et quatrième antigènes de contrôle, de façon à permettre une détection par lecture automatisée à l'aide d'une dite première et, le cas échéant, deuxième substance de détection, **caractérisée en ce que** l'on dépose de façon robotisée lesdits antigènes microbiens, de préférence corpusculaires, et dits antigènes de contrôle avec un robot de dépôt comprenant de préférence une seringue, lesdits antigènes corpusculaires étant de préférence associés à un colorant, de préférence encore un colorant fluorescent sous forme de suspension à une concentration permettant leur visualisation après dépôt à l'aide dudit colorant, et permettant ainsi de vérifier la fixation des dits antigènes sur ledit support solide.

28. Méthode selon la revendication 27, **caractérisée en ce que** l'on dépose de façon robotisée un dit antigène microbien et, le cas échéant, un dit deuxième antigène de contrôle et, le cas échéant, un dit quatrième antigène de contrôle, sous forme de suspension de corpuscules de cellules non confluentes, virus entiers ou bactéries entières ou fractions de cellules ou bactéries.

29. Méthode selon la revendication 28, **caractérisée en ce que** les antigènes de contrôle sous forme de suspension de cellules sont calibrés à une concentration de 10⁷ à 10⁹ cellules/ml, lesdits antigènes de contrôle ou antigènes microbiens sous forme de suspensions de bactéries ou fractions de bactéries sont calibrés à une concentration de 10⁷ à 10⁹ particules/ml et les suspensions de virus entiers à une concentration de 10⁹ à 10¹⁰ particules/ml.

30. Méthode selon l'une des revendications 28 ou 29, **caractérisée en ce que** l'on dépose lesdits antigènes de contrôle et microbiens corpusculaires en mélange avec un liant protéique, stabilisant la fixation sur ledit support solide.

31. Méthode selon la revendication 30, **caractérisée en ce que** ledit liant protéique est choisi parmi du jaune d'oeufs, de la gélatine, de l'albumine de sérum de bovin ou une IgG polyclonale non humaine, de préférence de chèvre.

32. Méthode selon la revendication 31, **caractérisée en ce que** ledit antigène microbien corpusculaire est déposé sur ledit support solide constitué d'une lame de verre, en mélange avec une immunoglobuline de type IgG polyclonale de chèvre.

33. Méthode selon l'une des revendications 27 à 32, **caractérisée en ce que** l'on réalise un lavage préalable dudit support solide avec une solution d'un mélange éthanol/acétone, de préférence à 50-50, puis on dépose et on stabilise la fixation des dits antigènes par adsorption physique sur ledit support solide par un traitement avec de l'alcool, de préférence méthanol ou éthanol, alcool que l'on élimine ensuite et, de préférence encore, on vérifie la fixation desdits antigènes par coloration, de préférence par un marquage fluorescent non spécifique des protéines ou de l'ADN.

34. Méthode selon l'une des revendications 27 à 32, **caractérisée en ce que** l'on complète la fixation par adsorption physique des dits antigènes de contrôle et microbiens par un traitement de réticulation, de préférence un traitement chimique par un agent bi-fonctionnel de couplage covalent.

## Claims

1. *In vitro* serological method for diagnosing microbial agents by immunodetection, wherein the presence is detected and, preferably, the quantity of patient immunoglobulins is assayed of both classes M and G, or only class G, specific to a microbial antigen characteristic of said microbial agent, in a patient's serum sample to be tested, by detection and quantification of an immunological reaction complex between said microbial antigen to be detected and a said specific class G immunoglobulin for IgG assay, or of class M for IgG assay, using a first detection substance constituted by an antibody reacting only with a said immunoglobulin of the patient species of class G for IgG assay, or a second detection substance constituted by an antibody reacting only with a said immunoglobulin of the patient species of class M for IgM assay, **characterized in that:**
1/ The steps are performed in which:
■ said serum sample to be tested then said first and second detection substances, or only said first detection substance is placed in contact with at least one solid support on which the following antigens have been attached:
- a first control antigen consisting of a non-specific class G immunoglobulin of the patient species, and
- a second control antigen containing DNA/histone complexes,
- optionally, a third control antigen consisting of a non-specific class M immunoglobulin of the patient species, the presence of said third control antigen being necessary in the event of IgM assay, and
- at least one said microbial antigen, and
■ a series of controls is conducted comprising:
a- controlling the reactivity of said second detection substance by verifying whether said first control antigen reacts with said second detection substance, and optionally control of the presence of rheumatoid factors in said serum sample by verifying whether the first control antigen reacts with said serum sample and said second detection substance, in the event of IgM assay,
b- controlling the presence of anti-nuclear antibodies in said serum sample to be tested by verifying whether said second control antigen reacts with said serum sample and the first detection substance,
c- controlling the reactivity of said second detection substance by verifying whether said third control antigen reacts with said second detection substance, in the event of IgM assay, and
d- controlling the presence of a serum of the patient species in the sample to be tested, and
2/ the reaction result between said microbial antigen, said serum sample and a said detection substance is taken into account only if the control of the presence of a serum of the patient species is positive and if the following accumulative conditions are met:
a- said first control antigen reacts with said first detection substance,
b- said second control antigen does not react, and
c- when applicable, said third control antigen reacts with said second detection substance, in the event of IgM assay.

2. The method as claimed in Claim 1, **characterized in that** a single solid support is used, contacted optionally simultaneously with said first and second detection substances containing a first and respectively a second labelling element, the second labelling element emitting a different signal to the first labelling element, said first and second detection substances containing a first and respectively a second antibody reacting only with a said immunoglobulin of the patient species of class G and respectively class M.

3. The method as claimed in Claim 1 or 2, **characterized in that** it is controlled such that said tested sample contains a serum of the patient species by detecting whether immunoglobulins of the patient species react with a fourth control antigen containing protein A of a *Staphylococcus aureus* bacterium, preferably said fourth antigen being a whole *Staphylococcus* bacterium, by contacting said sample with a solid support on which a said fourth control antigen is attached in the presence of said second detection substance which is an anti-immunoglobulin antibody of the patient species not reacting with said fourth control antigen, the control of the presence of a serum being positive if said fourth antigen reacts with said serum sample and said second detection substance.

4. The method as claimed in any of Claims 1 to 3, **characterized in that** said first detection substance is an animal immunoglobulin, preferably a goat or chicken immunoglobulin.

5. The method as claimed in any of Claims 1 to 4, **characterized in that,** optionally, the two said first and second detection substances are goat or chicken immunoglobulins, respectively anti-IgG and anti-IgM.

6. The method as claimed in any of Claims 1 to 5, **characterized in that** said second control antigen is constituted by human fibroblast cells.

7. The method as claimed in any of Claims 1 to 6, **characterized in that** said control antigens and microbial antigens are attached to the solid support by physical adsorption.

8. The method as claimed in any of Claims 1 to 7, **characterized in that** said microbial antigen is a corpuscular antigen consisting of a whole inactivated microbe or microbe fraction.

9. A serological diagnostic method as claimed in any of Claims 1 to 8, **characterized in that** said microbial agent is selected from microorganisms comprising a bacterium, a virus, a parasite or a fungus.

10. The serological diagnostic method as claimed in Claim 9, **characterized in that** said microbial antigen is an intracellular bacterium or a virus.

11. The serological diagnostic method as claimed in Claim 9 or 10, **characterized in that** said microbial antigen is selected from the bacteria of genus *Rickettsia, Coxiella, Bartonella, Tropheryma, Ehrlichia, Chlamydia, Mycoplasma, Treponema, Borrelia* and *Leptospira*.

12. The serological diagnostic method as claimed in Claim 11, **characterized in that** said microbial antigen is a bacterium responsible for endocarditis.

13. The serological diagnostic method as claimed in Claim 10, **characterized in that** said microbial antigen is a viral antigen selected from H.I.V, C.M.V., Epstein-Barr, Measles, Rubella, Hepatitis A and B viruses.

14. The method as claimed in any of Claims 1 to 13,
**characterized in that**:
- the detection and assay of the quantity of patient immunoglobulins of both classes M and G specific to a microbial antigen are performed,
- at least one said microbial antigen and said first, second and third and fourth control antigens are attached to one same solid support, and
- the same said first and second detection substances are used with different labelling elements for the detection of the different said microbial antigens, said first and second detection substances being animal immunoglobulins not reacting with said fourth antigen.

15. The method as claimed in any of Claims 1 to 14, **characterized in that** a glass or plastic slide is used as solid support, or a titre tube or well of a plastic microtitre plate.

16. The method as claimed in any of Claims 1 to 15, **characterized in that** in the sample to be tested the detection and, optionally the assay of said immunoglobulin of the patient species specific to said microbial antigen is performed, and the immunological reactions between said control antigens and said detection substances are read by automated reading using equipment for reading a fluorescent signal of a fluorescent substance corresponding to the labelling elements of said detection substances.

17. The method as claimed in any of Claims 1 to 16, **characterized in that** said microbial antigen is a vaccine antigen and said immunoglobulin specific to said vaccine agent to be detected is a class G immunoglobulin.

18. The method as claimed in Claim 17, **characterized in that** the vaccine status of a person is determined by detection, preferably quantification of IgG serum antibodies specific to the vaccine antigens of a plurality of pathogenic agents of bacterial, viral, fungal or parasitic type, by detecting, and preferably quantifying, an immunological reaction complex between each said vaccine antigen and respectively each said antibody specific to said vaccine antigen, which may be present in a human serum sample to be tested, comprising:
1. contacting one single, same said serum sample to be tested, then at least one said first detection substance reacting with at least one specific antibody and not reacting with any one of said vaccine antigens, with a same solid support on which a plurality of said vaccine antigens is attached corresponding to a plurality of pathogenic agents, and said first, second and preferably fourth control antigens, and
2. performing at least one said control of the reactivity of said first detection substance using a said first control antigen, and one said control of the presence of anti-nuclear antibodies using at least one said second control antigen, and controlling the presence of human serum in said sample to be tested.

19. The method as claimed in Claim 18, **characterized in that** said specific antibodies of IgG immunoglobulin type are detected and a said second detection substance is used which is an antiIgG immunoglobulin, consisting of a goat or chicken immunoglobulin.

20. The method as claimed in either of Claims 18 or 19, **characterized in that** said vaccine antigens are antigens of pathogenic agents selected from the viruses of mumps, rubella, measles, chicken pox, poliomyelitis, yellow fever, tick-borne encephalitis, hepatitis A, hepatitis B and the bacteria of *Bordetella pertussis*, tetanus and diphtheria.

21. The method as claimed in any of Claims 18 to 20, **characterized in that** it is determined whether the concentration of said specific antibodies reaches a threshold from which said specific antibody has a protective action protecting against the disease determined by the pathogen.

22. The method as claimed in any of Claims 18 to 21, **characterized in that** a determined volume of whole blood is collected using a capillary tube in a flask containing a determined volume of buffer allowing elution of the serum, the serum then preferably being diluted to a determined concentration, preferably 1:100 to 1:20.

23. The method as claimed in any of Claims 18 to 22, **characterized in that** for each detection and optionally quantification of a said vaccine antigen the following measurements are made:
1- a first measurement of a first value representing the quantity of a third labelling element, said first value being the intensity value of a signal emitted by said third fluorescent labelling element, said third labelling element binding itself non specifically to any protein in the depositing area of said vaccine antigen, and
2- a second measurement of a second value representing the quantity of a first labelling element emitting a different signal to said third labelling element, said second value being the intensity value of the signal emitted by this first fluorescent labelling element at a different excitation wavelength to that of said third fluorescent labelling element, said first labelling element being the labelling element of said first detection substance for said vaccine antigen in the depositing area of said antigen, and
3- the ratio between said first and second values is calculated, and
4- the value of said ratio is compared with the value of a reference ratio obtained with a collection of positive and negative reference sera, thereby making it possible to determine, by comparison, whether or not it is necessary to vaccinate the person against said vaccine antigen in relation to the ratio between said first and second values.

24. A diagnosis kit which can be used for implementing a method as claimed in any of Claims 1 to 23, wherein a IgG assay is performed, **characterized in that** it contains:
a- at least one said solid support on which are attached at least:
- one said microbial antigen such as defined in any of Claims 1 and 8 to 13 and 17, 18 and 20, and
- said first and second control antigens such as defined in either of Claims 1 and 6, and
- preferably a said fourth control antigen such as defined in Claim 3, and
b- a first detection substance such as defined in any one of Claims 1, 2, 4, 5 and 14.

25. The kit as claimed in Claim 24, which can be used for implementing a method as claimed in any of Claims 1 to 23, in which IgM assay is also performed, **characterized in that** it also contains:
a- one said solid support on which a said third control antigen such as defined in Claim 1 is also attached, and
b- a said second detection substance such as defined in any one of Claims 1, 2, 5 and 14.

26. The kit as claimed in Claim 24 or 25, **characterized in that** it comprises a same solid support on which are attached by physical adsorption at least one said corpuscular antigen and said control antigens.

27. A method for preparing a solid support of a diagnostic kit as claimed in any one of Claims 24 to 26, on which are attached at least one microbial antigen, and said first and second control antigen and preferably said third and fourth control antigens, enabling detection by automated reading using a said first and optionally said second detection substance, **characterized in that** said microbial antigens, preferably corpuscular, and control antigens are deposited by robot arrayer which preferably comprises a syringe, said corpuscular antigens preferably being associated with a dye, more preferably a fluorescent dye in the form of a suspension at a concentration enabling their visualization with said dye after being deposited, thereby making it possible to verify the attachment of said antigens to said solid support.

28. The method as claimed in Claim 27, **characterized in that** a robot arrayer is used to deposit a said microbial antigen and optionally a said second control antigen and optionally a said fourth control antigen in the form of a suspension of non-confluent cell corpuscles, whole viruses or whole bacteria, or fractions of cells or bacteria.

29. The method as claimed in Claim 28, **characterized in that** the control antigens in the form of a cell suspension are calibrated at a concentration of 10⁷ to 10⁹ cells/ml, said control antigens or microbial antigens in the form of suspensions of bacteria or bacteria fractions are calibrated at a concentration of 10⁷ to 10⁹ particles/ml, and the suspensions of whole viruses at a concentration of 10⁹ to 10¹⁰ particles/ml.

30. Method as in either of claims 28 or 29, **characterized in that** said control antigens and corpuscular microbial antigens are deposited in a mixture with a protein binder to stabilize attachment to said solid support.

31. The method as claimed in Claim 30, **characterized in that** said protein binder is selected from egg yolk, gelatine, bovine serum albumin or a non-human polyclonal IgG, preferably goat.

32. The method as claimed in Claim 31, **characterized in that** said corpuscular microbial antigen is deposited on said solid support consisting of a glass slide, in a mixture with an immunoglobulin of goat polyclonal IgG type.

33. The method as claimed in any of Claims 27 to 32, **characterized in that** prior washing of said solid support is performed with a solution of an ethanol/acetone mixture, preferably 50-50, then the said antigens are deposited and their attachment is stabilized by physical adsorption on said solid support by treatment with alcohol, preferably methanol or ethanol, which alcohol is subsequently removed, more preferably the attachment of said antigens is verified by staining, preferably with fluorescent labelling non-specific to the proteins or DNA.

34. The method as claimed in any of Claims 27 to 32, **characterized in that** the attachment by physical adsorption of said control antigens and microbial antigens is completed by cross-linking treatment, preferably chemical treatment using a bi-functional agent for covalent coupling.

## Patentansprüche

1. Verfahren zur serologischen In-vitro-Diagnostik eines mikrobiellen Agens mittels Immunnachweis, bei dem das Vorliegen von Patienten-Immunglobulinen der beiden Klassen M und G oder nur der Klasse G, die für ein für das mikrobielle Agens charakteristisches mikrobielles Antigen spezifisch sind, in einer zu untersuchenden Serumprobe des Patienten nachgewiesen wird und vorzugsweise deren Menge bestimmt wird, und zwar mittels Nachweis und Quantifizierung eines immunologischen Reaktionskomplexes zwischen dem nachzuweisenden mikrobiellen Antigen und einem spezifischen Immunglobulin der Klasse G für die quantitative IgG-Bestimmung oder der Klasse M für die quantitative IgM-Bestimmung, mit Hilfe einer ersten Nachweissubstanz bestehend aus einem lediglich mit einem Immunglobulin der Spezies des Patienten der Klasse G reagierenden Antikörper, für die quantitative IgG-Bestimmung, oder einer zweiten Nachweissubstanz bestehend aus einem nur mit einem Immunglobulin der Spezies des Patienten der Klasse M reagierenden Antikörper, für die quantitative IgM-Bestimmung, **dadurch gekennzeichnet, daß**
1/- die folgenden Schritte durchgeführt werden, bei denen:
• die zu untersuchende Serumprobe, anschließend die erste und die zweite Nachweissubstanz oder nur die erste Nachweissubstanz mit wenigstens einem festen Träger in Kontakt gebracht werden, auf dem die folgenden Antigene fixiert worden sind:
- ein erstes Kontrollantigen, das von einem unspezifischen Immunglobulin der Klasse G der Spezies des Patienten gebildet ist, und
- ein zweites Kontrollantigen, das DNA/Histon-Komplexe umfaßt, sowie
- gegebenenfalls ein drittes Kontrollantigen, das von einem unspezifischen Immunglobulin der Klasse M der Spezies des Patienten gebildet ist, wobei das Vorliegen des dritten Kontrollantigens im Fall einer quantitativen IgM-Bestimmung erforderlich ist, und
- wenigstens ein mikrobielles Antigen, und
• eine Reihe von Kontrollen durchgeführt wird, die folgendes umfassen:
a- die Kontrolle der Reaktivität der ersten Nachweissubstanz durch Überprüfen, ob das erste Kontrollantigen mit der ersten Nachweissubstanz reagiert, und - im Fall der quantitativen IgM-Bestimmung - die Kontrolle des Vorliegens von Rheumafaktoren in der Serumprobe durch Überprüfen, ob das erste Kontrollantigen mit der Serumprobe und der zweiten Nachweissubstanz reagiert,
b- die Kontrolle des Vorliegens antinuklearer Antikörper in der zu untersuchenden Serumprobe, durch Überprüfen, ob das zweite Kontrollantigen mit der Serumprobe und der ersten Nachweissubstanz reagiert,
c- im Fall der quantitativen IgM-Bestimmung, die Kontrolle der Reaktivität der zweiten Nachweissubstanz durch Überprüfen, ob das dritte Kontrollantigen mit der zweiten Nachweissubstanz reagiert, und
d- die Kontrolle des Vorliegens eines Serums der Spezies des Patienten in der zu untersuchenden Probe, und
2/- das Ergebnis einer Reaktion zwischen dem mikrobiellen Antigen, der Serumprobe und einer Nachweissubstanz nur berücksichtigt wird, wenn die Kontrolle des Vorliegens eines Serums der Spezies des Patienten positiv ist und wenn die folgenden kumulativen Bedingungen gemeinsam vorliegen:
a- das erste Kontrollantigen reagiert mit der ersten Nachweissubstanz,
b- das zweite Kontrollantigen reagiert nicht, und
c- gegebenenfalls, im Fall der quantitativen IgM-Bestimmung, reagiert das dritte Kontrollantigen mit der zweiten Nachweissubstanz.

2. Verfahren nach Anspruch 1, **dadurch gekennzeichnet, daß** ein einziger fester Träger verwendet wird, der gegebenenfalls gleichzeitig mit der ersten und der zweiten Nachweissubstanz, die ein erstes bzw. ein zweites Markierungselement enthalten, in Kontakt gebracht wird, wobei das zweite Markierungselement ein anderes Signal als das erste Markierungselement aussendet, wobei die erste und die zweite Nachweissubtanz einen ersten bzw. einen zweiten Antikörper enthalten, der nur mit einem Immunglobulin der Spezies des Patienten der Klasse G bzw. der Klasse M reagiert.

3. Verfahren nach Anspruch 1 oder 2, **dadurch gekennzeichnet, daß** kontrolliert wird, ob die untersuchte Probe tatsächlich ein Serum der Spezies des Patienten enthält, indem nachgewiesen wird, ob Immunglobuline der Spezies des Patienten mit einem vierten Kontrollantigen, welches das Protein A eines Staphylococcus aureus-Bakteriums enthält, reagieren, wobei vorzugsweise das vierte Antigen ein vollständiges Staphylococcus-Bakterium ist, und zwar **dadurch**, daß die Probe in Anwesenheit der ersten Nachweissubstanz, die ein Anti-Immunglobulin-Antikörper der Spezies des Patienten ist, der nicht mit dem vierten Kontrollantigen reagiert, mit einem festen Träger, auf dem ein viertes Kontrollantigen fixiert ist, in Kontakt gebracht wird, wobei die Kontrolle des Vorliegens eines Serums positiv ist, wenn das vierte Antigen mit der Serumprobe und der ersten Nachweissubstanz reagiert.

4. Verfahren nach einem der Ansprüche 1 bis 3, **dadurch gekennzeichnet, daß** die erste Nachweissubstanz ein tierisches Immunglobulin, vorzugsweise ein Immunglobulin von der Ziege oder vom Huhn ist.

5. Verfahren nach einem der Ansprüche 1 bis 4, **dadurch gekennzeichnet, daß** gegebenenfalls beide Nachweissubtanzen, also die erste und die zweite, Immunglobuline von der Ziege oder vom Huhn, Anti-IgG bzw. Anti-IgM sind.

6. Verfahren nach einem der Ansprüche 1 bis 5, **dadurch gekennzeichnet, daß** das zweite Kontrollantigen aus Zellen humaner Fibroblasten besteht.

7. Verfahren nach einem der Ansprüche 1 bis 6, **dadurch gekennzeichnet, daß** die Kontrollantigene und mikrobiellen Antigene durch physikalische Adsorption auf dem festen Träger fixiert sind.

8. Verfahren nach einem der Ansprüche 1 bis 7, **dadurch gekennzeichnet, daß** das mikrobielle Antigen ein korpuskuläres Antigen ist, das aus einer inaktivierten vollständigen Mikrobe oder einer Mikrobenfraktion besteht.

9. Serologisches Diagnostikverfahren nach einem der Ansprüche 1 bis 8, **dadurch gekennzeichnet, daß** das mikrobielle Agens aus den Mikroorganismen umfassend ein Bakterium, ein Virus, einen Parasiten oder einen Pilz ausgewählt ist.

10. Serologisches Diagnostikverfahren nach Anspruch 9, **dadurch gekennzeichnet, daß** das mikrobielle Antigen ein intrazelluläres Bakterium oder ein Virus ist.

11. Serologisches Diagnostikverfahren nach Anspruch 9 oder 10, **dadurch gekennzeichnet, daß** das mikrobielle Antigen aus den Bakterien der Gattung Rickettsia, Coxiella, Bartonella, Tropheryma, Ehrlichia, Chlamydia, Mycoplasma, Treponema, Borrelia und Leptospira ausgewählt ist.

12. Serologisches Diagnostikverfahren nach Anspruch 11, **dadurch gekennzeichnet, daß** das mikrobielle Antigen ein für eine Endokarditis verantwortliches Bakterium ist.

13. Serologisches Diagnostikverfahren nach Anspruch 10, **dadurch gekennzeichnet, daß** das mikrobielle Antigen ein virales Antigen ist, welches aus den Viren H.I.V., C.M.V., dem Epstein-Barr-Virus, Masernvirus, Rubella-Virus, Hepatitis-A-und Hepatitis-B-Virus ausgewählt ist.

14. Verfahren nach einem der Ansprüche 1 bis 13, **dadurch gekennzeichnet, daß**:
- die Menge der Immunglobuline von Patienten der beiden Klassen M und G, die für ein mikrobielles Antigen spezifisch sind, nachgewiesen und bestimmt wird,
- wenigstens ein mikrobielles Antigen sowie das erste, das zweite und das dritte und das vierte Kontrollantigen auf einem gleichen festen Träger fixiert werden, und
- für den Nachweis der unterschiedlichen mikrobiellen Antigene die gleichen ersten und zweiten Nachweissubstanzen mit unterschiedlichen Markierungselementen verwendet werden, wobei die erste und die zweite Nachweissubstanz tierische Immunglobuline sind, die nicht mit dem vierten Antigen reagieren.

15. Verfahren nach Anspruch 1 bis 14, **dadurch gekennzeichnet, daß** als fester Träger ein Objektträger aus Glas oder Kunststoff, ein Titrationsröhrchen oder ein Well einer Kunststoff-Mikrotiterplatte verwendet wird.

16. Verfahren nach einem der Ansprüche 1 bis 15, **dadurch gekennzeichnet, daß** durch automatisiertes Lesen mit Hilfe eines Gerätes zum Lesen eines fluoreszierenden Signals einer den Markierungselementen der Nachweissubstanzen entsprechenden fluoreszierenden Substanz das für das mikrobielle Antigen spezifische Immunglobulin der Spezies des Patienten in der zu untersuchenden Probe sowie die immunologischen Reaktionen zwischen den Kontrollantigenen und den Nachweissubstanzen nachgewiesen werden und gegebenenfalls die Menge des genannten Immunglobulins in der zu untersuchenden Probe bestimmt wird.

17. Verfahren nach einem der Ansprüche 1 bis 16, **dadurch gekennzeichnet, daß** das mikrobielle Antigen ein Impfantigen ist und das für das nachzuweisende Impfantigen spezifische Immunglobulin ein Immunglobulin der Klasse G ist.

18. Verfahren nach Anspruch 17, **dadurch gekennzeichnet, daß** der Impfstatus eines Individuums mittels Nachweis, vorzugsweise Quantifizierung der IgG-Serumantikörper, die für Impfantigene einer Vielzahl pathogener Agenzien von der Art Bakterien, Viren, Pilzen oder Parasiten spezifisch sind, **dadurch** bestimmt wird, daß der Nachweis, und vorzugsweise die Quantifizierung, eines Komplexes immunologischer Reaktionen zwischen jedem Impfantigen und jeweils jedem für das Impfantigen spezifischen, eventuell in einer zu untersuchenden Probe eines humanen Serums vorliegenden Antikörper durchgeführt wird, umfassend:
1- das Inkontaktbringen einer gleichen zu untersuchenden Serumprobe, anschließend wenigstens einer ersten Nachweissubstanz, die mit wenigstens einem spezifischen Antikörper reagiert und die nicht mit irgendeinem der Impfantigene reagiert, mit einem gleichen festen Träger, auf dem eine Vielzahl von Impfantigenen, die einer Vielzahl pathogener Agenzien entsprechen, sowie das erste, das zweite und vorzugsweise das vierte Kontrollantigen fixiert sind, und
2- wenigstens eine Kontrolle der Reaktivität der ersten Nachweissubstanz mit Hilfe eines ersten Kontrollantigens sowie eine Kontrolle des Vorliegens antinuklearer Antikörper mit Hilfe wenigstens eines zweiten Kontrollantigens und die Kontrolle des Vorliegens eines humanen Serums in der zu untersuchenden Probe.

19. Verfahren nach Anspruch 18, **dadurch gekennzeichnet, daß** spezifische Antikörper des Typs IgG-Immunglobuline nachgewiesen werden und eine erste Nachweissubstanz eingesetzt wird, die ein Anti-IgG-Immunglobulin ist, das von einem Immunglobulin von der Ziege oder vom Huhn gebildet ist.

20. Verfahren nach einem der Ansprüche 18 oder 19, **dadurch gekennzeichnet, daß** die Impfantigene Antigene der pathogenen Agenzien ausgewählt unter den folgenden sind, nämlich dem Mumpsvirus, Rubella-Virus, Masernvirus, Varizella-Virus, Poliomyelitis-Virus, Gelbfieber-Virus, Meningo-Enzephalitis-Virus, Hepatitis-A-Virus, Hepatitis-B-Virus und den Keuchhusten-Bakterien Bordetella pertussis, den Tetanus-und Diphterie-Bakterien.

21. Verfahren nach einem der Ansprüche 18 bis 20, **dadurch gekennzeichnet, daß** ermittelt wird, ob die Konzentration des spezifischen Antikörpers eine Schwelle erreicht, ab welcher der spezifische Antikörper eine Schutzwirkung aufweist, die gegen die durch das Pathogen bestimmte Krankheit schützt.

22. Verfahren nach einem der Ansprüche 18 bis 21, **dadurch gekennzeichnet, daß** eine bestimmte Gesamtblutmenge mit Hilfe einer Kapillarröhre in einem Kolben gesammelt wird, der eine bestimmte Menge eines Puffers enthält, der das Eluieren des Serums ermöglicht, wobei das Serum dann auf eine bestimmte Konzentration, vorzugsweise zwischen 1/100 und 1/20 verdünnt wird.

23. Verfahren nach einem der Ansprüche 18 bis 22, **dadurch gekennzeichnet, daß** für jeden Nachweis und gegebenenfalls jede Quantifizierung eines Impfantigens die folgenden Messungen durchgeführt werden:
1- eine erste Messung eines ersten Wertes, der für die Menge eines dritten Markierungselements repräsentativ ist, wobei der erste Wert der Wert der Stärke eines durch das dritte fluoreszierende Markierungselement ausgesandten Signals ist, wobei das dritte Markierungselement auf nicht spezifische Weise an jedes Protein im Auftragsbereich des Impfantigens bindet, und
2- eine zweite Messung eines zweiten Wertes, der für die Menge eines ein anderes Signal als das dritte Markierungselement aussendenden ersten Markierungselements repräsentativ ist, wobei der zweite Wert der Wert der Stärke des Signals ist, das durch dieses erste fluoreszierende Markierungselement bei einer anderen Anregungswellenlänge als der des dritten fluorszierenden Markierungselements ausgesendet wird, wobei das erste Markierungselement das Element des Markierungselements der ersten Nachweissubstanz des Impfantigens im Auftragsbereich des Antigens ist, und
3- es wird das Verhältnis zwischen dem ersten und dem zweiten Wert berechnet, und
4- es wird der Wert des Verhältnisses mit dem eines mit einer Sammlung positiver und negativer Referenzseren erhaltenen Referenzverhältnisses verglichen, wodurch es mittels Vergleich möglich ist, entsprechend dem Wert des Verhältnisses zwischen dem ersten und dem zweiten Wert die Notwendigkeit oder die Nichtnotwendigkeit des Impfens der Person für das Impfantigen zu bestimmen.

24. Diagnose-Kit, das für die Durchführung eines Verfahrens nach einem der Ansprüche 1 bis 23 nützlich ist, bei dem die quantitative IgG-Bestimmung durchgeführt wird, **dadurch gekennzeichnet, daß** es folgendes umfaßt:
a- wenigstens einen festen Träger auf dem wenigstens folgendes fixiert ist:
- ein mikrobielles Antigen, wie es in einem der Ansprüche 1 und 8 bis 13 sowie 17, 18 und 20 definiert ist, und
- ein erstes sowie ein zweites Kontrollantigen, wie sie in einem der Ansprüche 1 und 6 definiert sind, und
- vorzugsweise ein viertes Kontrollantigen, wie es in Anspruch 3 definiert ist, und
b- eine erste Nachweissubstanz, wie sie in einem der Ansprüche 1, 2, 4, 5 und 14 definiert ist.

25. Kit nach Anspruch 24, das für die Durchführung eines Verfahrens nach einem der Ansprüche 1 bis 23 nützlich ist, bei dem ferner die quantitative IgM-Bestimmung durchgeführt wird, **dadurch gekennzeichnet, daß** es außerdem folgendes umfaßt:
a- einen festen Träger, auf dem ferner ein drittes Kontrollantigen, wie es in Anspruch 1 definiert ist, fixiert ist, sowie
b- eine zweite Nachweissubstanz, wie sie in einem der Ansprüche 1, 2, 5 und 14 definiert ist.

26. Kit nach Anspruch 24 oder 25, **dadurch gekennzeichnet, daß** es einen gleichen festen Träger umfaßt, auf dem mittels physikalischer Adsorption wenigstens ein korpuskuläres Antigen und die Kontrollantigene fixiert sind.

27. Verfahren zum Herstellen eines festen Trägers eines Diagonse-Kits nach einem der Ansprüche 24 bis 26, auf dem wenigstens ein mikrobielles Antigen sowie ein erstes und ein zweites Kontrollantigen sowie vorzugsweise ein drittes und ein viertes Kontrollantigen fixiert sind, so daß ein Nachweis durch automatisiertes Lesen mit Hilfe einer ersten und gegebenenfalls zweiten Nachweissubstanz ermöglicht wird, **dadurch gekennzeichnet, daß** die mikrobiellen, vorzugsweise korpuskulären Antigene sowie Kontrollantigene mittels eines vorzugsweise eine Spritze umfassenden Auftragsroboters aufgetragen werden, wobei die korpuskulären Antigene vorzugsweise einem Farbstoff zugeordnet sind, weiterhin vorzugsweise einem fluoreszierenden Farbstoff in Form einer Suspension in einer Konzentration, die deren Visualisierung nach dem Auftragen mit Hilfe des Farbstoffs ermöglicht, wodurch es möglich ist, die Fixierung der Antigene auf dem festen Träger zu überprüfen.

28. Verfahren nach Anspruch 27, **dadurch gekennzeichnet, daß** ein mikrobielles Antigen und gegebenenfalls ein zweites Kontrollantigen sowie gegebenenfalls ein viertes Kontrollantigen, in Form einer Suspension von Korpuskeln nicht konfluenter Zellen, ganzen Viren oder ganzen Bakterien oder Zell- oder Bakterienfraktionen mittels Roboter aufgetragen wird.

29. Verfahren nach Anspruch 28, **dadurch gekennzeichnet, daß** die Kontrollantigene in Form einer Zellsuspension auf eine Konzentration von 10⁷ bis 10⁹ Zellen/ml eingestellt werden, die Kontrollantigene oder mikrobiellen Antigene in Form von Suspensionen von Bakterien oder Bakterienfraktionen auf eine Konzentration von 10⁷ bis 10⁹ Partikel/ml eingestellt werden und die Suspensionen von ganzen Viren auf eine Konzentration von 10⁹ bis 10¹⁰ Partikel/ml eingestellt werden.

30. Verfahren nach einem der Ansprüche 28 oder 29, **dadurch gekennzeichnet, daß** die Kontrollantigene und korpuskulären mikrobiellen Antigene in Mischung mit einem Proteinbindemittel, das die Fixierung auf dem festen Träger stabilisiert, aufgetragen werden.

31. Verfahren nach Anspruch 30, **dadurch gekennzeichnet, daß** das Proteinbindemittel aus Eigelb, Gelatine, Rinderserumalbumin oder einem polyklonalen nicht humanen IgG, vorzugsweise von der Ziege ausgewählt ist.

32. Verfahren nach Anspruch 31, **dadurch gekennzeichnet, daß** das korpuskuläre mikrobielle Antigen auf dem von einem gläsernen Objektträger gebildeten festen Träger in Mischung mit einem Immunglobulin von der Art polyklonales IgG von der Ziege aufgetragen wird.

33. Verfahren nach einem der Ansprüche 27 bis 32, **dadurch gekennzeichnet, daß** ein vorheriges Waschen des festen Trägers mit einer Lösung aus einem Ethanol/Aceton-Gemisch, vorzugsweise in einem Verhältnis 50/50, durchgeführt wird, dann aufgetragen wird und die Fixierung der Antigene mittels physikalischer Adsorption auf dem festen Träger durch eine Behandlung mit Alkohol, vorzugsweise Methanol oder Ethanol stabilisiert wird, Alkohohl, der anschließend entfernt wird, und weiterhin vorzugsweise die Fixierung der Antigene mittels Anfärben, vorzugsweise durch eine unspezifische fluoreszierende Markierung der Proteine oder der DNA überprüft wird.

34. Verfahren nach einem der Ansprüche 27 bis 32, **dadurch gekennzeichnet, daß** die Fixierung mittels physikalischer Adsorption der Kontrollantigene und mikrobiellen Antigene durch eine Vernetzungsbehandlung, vorzugsweise eine chemische Behandlung mit einem bifunktionellen Agens zur kovalenten Kopplung komplettiert wird.
